# EUROPEAN PATENT SPECIFICATION

(11) **EP 3 436 021 B1**
(45) Date of publication and mention of the grant of the patent: **29.07.2026**
(21) Application number: 17824658.3
(22) Date of filing: 25.03.2017
(51) Int. Cl.: A61K 31/4245, A61K 31/535, C07D 265/00, C07D 271/00, A61K 45/06, A61P 25/00, A61K 31/5377, C07D 413/06, C07D 413/12

(54) **COMPOSITIONS COMPRISING LOW IMPACT AMPAKINES FOR TREATING ATTENTION DEFICIT DISORDERS**
ZUSAMMENSETZUNGEN MIT NIEDRIG WIRKENDEN AMPAKINEN ZUR BEHANDLUNG VON AUFMERKSAMKEITSDEFIZITSTÖRUNGEN
COMPOSITIONS COMPRENANT DES AMPAKINES A FAIBLE IMPACT POUR TRAITER DES TROUBLES DE DÉFICIT DE L'ATTENTION

(30) Priority: 29.03.2016 US 201662314869 P
(43) Date of publication of application: 06.02.2019
(73) Proprietor: RespireRx Pharmaceuticals, Inc., Glen Rock, NJ 07452 (US); Lippa, Arnold, Stan, Ridgewood, NJ 07650-4726 (US)
(72) Inventor: LIPPA, Arnold, Stan, Ridgewood, NJ 07650-4726 (US)
(74) Representative: Patentree
(86) International application number: PCT/US2017/024188
(87) International publication number: WO 2018/009256

(56) References cited:
- WO-A1-2005/051389
- WO-A1-2008/143963
- WO-A2-2009/023126
- US-A1- 2005 096 395
- US-A1- 2005 228 019
- US-A1- 2010 069 377
- US-A1- 2011 003 835
- US-A1- 2012 101 073
- US-A1- 2014 024 650
- US-A1- 2014 100 249
- US-A1- 2014 315 805
- DANYSZ W: "CX-516 CORTEX PHARMACEUTICALS", CURRENT OPINION IN INVESTIGATIONAL DRUGS, PHARMAPRESS, US, vol. 3, no. 7, 1 January 2002 (2002-01-01), pages 1081 - 1088, XP008093723, ISSN: 1472-4472
- ANONYMOUS ET AL: "Cortex Pharmaceuticals, Inc. To Present Phase II Data From Adult ADHD Study At AACAP Meeting", 5 September 2006 (2006-09-05), XP093064291, Retrieved from the Internet <URL:https%3A%2F%2Fwww.biospace.com%2Farticle%2Freleases%2Fcortex-pharmaceuticals-inc-to-present-phase-ii-data-from-adult-adhd-study-at-aacap-meeting-%2F%3Fs%3D106> [retrieved on 20230715]
- GAINETDINOV R. R. ET AL: "Glutamatergic modulation of hyperactivity in mice lacking the dopamine transporter", PROCEEDINGS OF THE NATIONAL ACADEMY OF SCIENCES, vol. 98, no. 20, 25 September 2001 (2001-09-25), pages 11047 - 11054, XP055881043, ISSN: 0027-8424, Retrieved from the Internet <URL:https://www.pnas.org/content/pnas/98/20/11047.full.pdf> [retrieved on 20220119], DOI: 10.1073/pnas.191353298
- FRANKE A. ET AL: "Psychostimulantien und Nicht-Stimulantien in der heutigen und zukünftigen ADHS-Therapie", FORTSCHRITTE DER NEUROLOGIE PSYCHIATRIE, vol. 80, no. 03, 1 March 2012 (2012-03-01), DE, pages 130 - 140, XP093064292, ISSN: 0720-4299, Retrieved from the Internet <URL:http://dx.doi.org/10.1055/s-0031-1273223> [retrieved on 20230715], DOI: 10.1055/s-0031-1273223
- FROESTL ET AL.: "Cognitive Enhancers (Nootropics). Part 1: Drugs Interacting with Receptors", JOURNAL OF ALZHEIMER'S DISEASE, vol. 32, no. 4, 14 November 2012 (2012-11-14), pages 793 - 887, XP055580011, DOI: 10.3233/JAD-2012-121186

## Description

### TECHNICAL FIELD

The present invention relates to composition comprising one or more ampakine for use in treating attention deficit disorders, including attention deficit hyperactivity disorder (ADHD). The use comprising administering to the subject the one or more ampakines in an amount effective to alleviate one or more symptom(s) of ADD in the subject, wherein the ampakine is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739).

### BACKGROUND OF THE INVENTION

Attention deficit disorders (ADDs) principally present as Attention-deficit hyperactivity disorder (ADHD), a central nervous system disorder characterized by developmentally inappropriate impusivity, inattention, and hyperactivity, among other symptoms. ADHD is one of the most prevalent developmental disorders in children, with an incidence ranging between 5-10% (Polanczyk *et al.,* 2007). ADHD was once regarded a childhood disorder, however it is now recognized that ADHD often continues through adolescence and into adulthood. Approximately 3-4% or more of the adult population has continuing ADHD (Kessler *et al.,* 2006; Faraone and Biederman, 2005). Major symptoms of ADHD in adults include inattention, disorganization, lack of concentration and impulsivity, often associated with reduced cogntive functioning, low educational attainment, poor vocational achievement , and problems with social and family relations (Biederman *et al.,* 2006; Barkely *et al.,* 2006).

The fundamental causes of ADHD are not known. Dysfunction of the prefrontal cortex and related neurophysiology has been proposed as a principal deficit in ADHD (Arnsten, 2009). Consistent with this theory, Arnsten and others report that abnormal catecholaminergic functions in the prefrontal cortex are associated with ADHD. Yet much remains to be determined regarding the neurophysiologic mechanisms, neurochemical factors and molecular targets for effective clinical management of ADHD.

Pharmacotherapy is the primary treatment for ADHD. Stimulants such as methylphenidate and amphetamines are the most common class of drug used to reduce symptoms of ADHD. The primary mechanism of action for these stimulants is to inhibit dopamine and norepinephrine neurotransporters. While these stimulants are reported to be effective against core symptoms of ADHD (with reported response rates as high as 70% Spencer *et al.,* 2005), use of these stimulants is attended by a high risk of abuse and dependency, as well as diversion and neurotoxic effects in the case of amphetamines (Berman *et al.,* 2009). Abuse risks for ADHD stimulants is heightened in adults, by a frequent co-occurrence of substance abuse in adults with ADHD (Levin and Kleber, 1995; Ohlmeier, 2008). Other side effects of these drugs relate directly to the stimulant activity of the drugs, which may cause nervousness, headache, upset stomach, anxiety, insomnia, appetite suppression, elevated blood pressure and motor tics. Many of these side effects are particularly concerning for pediatric patients.

Certain non-stimulate drugs have been proposed for treating ADHD, perhaps the most successful of which is atomoxetine (a selective norepinephrine reuptake inhibitor). The advantages of atomoxetine compared to stimulants includes avoidance of adverse stimulation effects, and avoidance of abuse potential. Atomoxetine also confers an added advantage of treating co-morbid anxiety and depression. However, atomoxetine has limited efficacy for ADHD alone, and the drug takes 2-4 weeks for clinical benefits to be observed (Spencer *et al.,* 1998; Newcorn *et al.,* 2008).

Accordingly, there is an urgent need in the art for more effective ADHD medications which will confer improved efficacy and minimize adverse side effects including those that attend the use of current, stimulant drugs for ADHD.

Following the discovery twenty-five years ago of the molecular structure of the AMPA glutamate receptor (hereafter "AMPA receptor"), various compounds have been explored for their potential to interact with AMPA receptors. Early investigations to identify such compounds produced a host of early drug candiates, including aniracetam, benzothiazides, diazoxide and cyclothiazide. These early drug candidates were reported to exhibit a range of potentially useful biological activities in animal models. Unfortunately, these drug candiates have generally been determined to have limited clinical utility. In particular, benzothiazides were initially reported to have potent neurologic effect in certain animal models, but therapeutic use of these compounds was limited by undesirable side effects. In contrast, aniracetam was limited in therapeutic development by low potency and rapid metabolism. Neither aniracetam nor early thiadiazides yielded successful clinical drugs for modulation AMPA receptor activities.

Following these early unsuccessful drug development efforts, advanced chemical investigations were undertaken by Cortex Pharmaceuticals and Lilly Research Labs. These extensive research efforts led to the identification of new classes of chemicla modulators of AMPA receptor function. Cortex scientists developed a novel family of positive allosteric modulators of the AMPA glutamate receptors, termed "ampakines." Lilly scientists independently developed a series of distinct AMPA receptor modulator candidates, biarylpropylsulfonamides. The Cortex ampakines and Lilly biarylpropylsulfonamides exhibited certain pharmacological similarities in their effects on receptor bio-physics and synaptic transmission (Arai et al., 1994, 1996a,b, 2000), gene expression (Holst et al., 1998; Lauterborn et al., 2000), and neural activity (Staubli et al., 1994a,b; Hampson et al., 1998).

In other studies these distinct classes of AMPA receptor modulating compounds showed certain similarities in activity in models of animal as well as human behavior (e.g., Granger et al., 1993; Larson et al., 1995, 1996; Lynch et al., 1996; Goff et al., 2001). Briefly, both classes bound to the cyclothiazide binding site, reduced the rate of AMPA receptor desensitization, stimulated production of BDNF and enhanced synaptic transmission and long term potentiation. With regard to behavioral effects, each of these classes of compounds exhibited activity in certain animal models of neural activity, including cognition. Unfortunately, these early drug candidates were generally unsuited as as clinical candidates for their propensity to produce convulsions at therapeutic dose range.

In 2002, Cortex scientists reported identification and partial characterization of two novel benzoylpiperidine ampakines ("CX516" and "CX546"-see below), which exhibited significantly different properties. CX546 (a "Type 1 ampakine") binds to the cyclothiazide binding site, prolongs deactivation and inhibits desensitization in excised hippocampal patches. Associated with these activities, CX546 prolongs synaptically evoked response duration using whole-cell recordings from CA1 pyramidal neurons of hippocampal slices. In contrast, CX516 (a "Type 2 ampakine"), does not bind to the cyclothiazide binding site, primarily increases amplitude much more so than it prolongs deactivation, and inhibits desensitization in excised hippocampal patches and increases much more so than prolongs synaptic responses. Based on kinetic receptor modeling, Cortex scientists concluded that CX546 and related Type 1 ampakines mainly slow channel closing, while CX516 and other Type 2 ampakines preferentially accelerate channel opening. Highlighting the differences between these two groups of compounds are observations that CX546 and otheR Type 1 ampakines produce generalized discharges in hippocampal slices, while CX516 and other Type 2 ampakines do not.

Subsequent studies by Cortex researchers suggested that, despite differences in their molecular mechanisms of action, Type 1 and Type 2 ampakines can display similar behavioral effects in animal models of cognition and depression. Behavioral effects of CX516 were studied intensively. Despite having a relatively short half-life, CX516 was investigated primary clinical studies to evaluate its memory enhancing potential and prospective ability to alleviate symptoms of schizophrenia and Fragile X Syndrome (FXS). When administered to healthy young male volunteers, CX516 was initially reported to significantly improve delayed retention of visual material and ability to identify difficult odors, in addition to enhancing learning of a visuospatial maze (Ingvar et al, 1997). Later studies were directed toward the potential use of CX516 as an add-on to clozapine for treating schizophrenia. These studies eployed higher ampakine doses, and the patients receiving CX516 reportedly showed improved scores in attention and item acquisition tests, with some differences in distraction and complex figure tests. Two weeks after completing this study, subjects who received the ampakine also reportedly showed significant between-group difference in a distraction test and five-item acquisition test, and moderate effect sizes on measures of attention and verbal fluency (Goff et al, 2001). Further analysis of this data determined that patients receiving CX516 were substantially more impaired at baseline than the placebo group, raising uncertainty regarding how to interpret the repoted activities of CX516 in the experimental group (Goff et al, 2001).

An add-on trial was performed by Cortex a few years later with CX516 added onto treatment of schizophrenia patients with clozapine, olanzapine or risperidone (Goff et al, 2008). Experimental subjects were given CX516 for four weeks and were tested at weeks 0, 4 and 8 (4 weeks after treatment completion). In this larger study, subjects receiving CX516 did not significantly differ in cognition tests from placebo groups at weeks 4 or 8 (Goff et al, 2008). Additionally, CX516 treatment was associated with unacceptable adverse side effects of fatigue, epigastric discomfort and insomnia. CX516 was therefore not determined to be effective in this larger cohort, either for improving cognition or symptoms commonly associated with schizophrenia when added to antipsychotic drugs (Goff et al, 2008).

CX516 was further evaluated for its potential in treating fragile X syndrome (FXS). Patients receiving CX516 did not exhibit improved memory, enhanced language use, or improved attention, executive functioning or overall functioning (Berry-Kravis et al, 2006). Based on these and related study findings, Cortex determined that it's most favorable drug candidate for cognitive therapeutic uses, CX516, was ineffective and unsuited for this field of clinical use.

The foregoing efforts by Cortex, Lilly and others, which have spanned two decades of research to develop AMPA receptor modulating drugs to treat cognitive and psychiatric disorders, have all been equally disappointing and unsuccessful. Despite promising early stage development results by Cortex and Lilly, no clinically successful drug candidate has emerged from among large, discrete classes of AMPA modulator compounds, for treating any category of cognitive disorder. Leading this campaign with ground-breaking efforts, Cortex was unable to find clinical utility for its lead Type 2 ampakine drug candidate, CX516, for any cognitive or psychiatric condition, including ADHD, schizophrenia or FXS.

Thus, with ever-increasing diagnoses of ADHD and other cognitive and psychiatric disorders, there remains an urgent and growing need in the art for new, more effective drugs to treat these conditions. This unmet goal embraces a need for further exploration to identify drugs that can selectively modulate AMPA receptor functions implicated in these conditions, within therapeutically useful and clinically acceptable compositions and methods.

A related need exists for new methods and compositions employing ampakines (positive allosteric modulators of AMPA glutamate receptors), to effectively modulate AMPA receptor activities to yield therapeutic effects in patients with ADHD and other cogntitive disorders, without imposing unacceptable adverse side effects.

Document WO2009/023126 A2 discloses low impact bicyclic amide ampakines for the treatment of ADHD.

Document WO 2008/143963 A1 discloses low impact disubstituted amide ampakines such as carboxamides for the treatment of ADHD.

Document WO 2005/051389 A1 discloses use of (+)-isopropyl 2-methoxyethyl 4-(2-chloro-3-cyano-phenyl)-1,4-dihydro-2,6-dimethyl-pyridine-3,5-dicarboxylate, as a sole active agent or in combination with amakines for the treatment of ADHD and ADD.

Document US 2005/228019 A1 discloses use of AMPAkines such as CX717 for the treatment of ADD in combination with an acetylcholinesterase inhibitor.

Document DANYSZ W "CX-516 CORTEX PHARMACEUTICALS", CURRENT OPINION IN INVESTIGATIONAL DRUGS, PHARMAPRESS, US, vol. 3, no.7, 1 January 2002 discloses ampakine CX-516 (ampalex) in phase clinical study has an effect against ADHD.

Document US 2014/024650 A1 discloses a method for treating an attention deficit disorder in a mammalian subject comprising administering to the subject an anti-ADD ampakine in an amount effective to treat ADD in the subject.

Document US 2005/096395 A1 discloses the symptoms of ADHD and methods for treating them. Rao identifies the multiple symptoms and rating scale to assess the efficacy of the compounds.

Document US 2014/315805 A1 discloses anti-ADD ampakine, a low impact anti-ADHD disubstituted amide ampakine selected from N-methyl-N-tetrahydro-2H-pyran-4-yl[2,1,3]-benzoxadiazole-5-carboxamide (para [0026], 'CX-1739').

Document FROESTL et al.: "Cognitive Enhancers (Nootropics). Part 1: Drugs Interacting with Receptors", JOURNAL OF ALZHEIMER'S DISEASE, vol. 32, no. 4, 14 November 2012 discloses
Document US 2012/101073 A1 discloses the ampakine CX1763.

Document US 2011/003835 A1 discloses discloses an ampakine as a bicyclic amide ampakine selected from 1-azabicyclo[2.2.1]hept-1-yl([2.1.3]-benzoxadiazol-5-yl)methanone or 2-azabicyclo[2.2.1]hept-5-en-2-yl([2.1.3]-benzoxadiazol-5-yl)methanone; (Claim 46, second compound).

Document US 2014/100249 A1 discloses ampakines CX-1739, CX-2076, CX-516, CX-546, CX-614, CX-717 used in combination with an amphetamine or a methylphenidate for the treatment of ADD or ADHD.

Document US 2010/069377 A1 discloses that ampakine compounds do not exhibit convulsant activity in a subject.

Document Anonymous ET AL: "Cortex Pharmaceuticals, Inc. To Present Phase II Data From Adult ADHD Study At AACAP Meeting", 5 September 2006 discloses phase Ila clinical trial of CX717 in adults with attention deficit/ hyperactivity disorder (ADHD) and reports that CX717 at a dose of 800 mg twice a day was significantly more effective than placebo on the total ADHD Rating Scale and on both the hyperactivity and inattentiveness subscales.

Document Franke A. ET AL: "Psychostimulantien und Nicht-Stimulantien in der heutigen und zukunftigen ADHS-Therapie", FORTSCHRITTE DER NEUROLOGIE PSYCHIATRIE, vol. 80, no. 03, 1 March 2012 discloses CX717 and CX1739.

### SUMMARY OF EXEMPLARY EMBODIMENTS

The invention fulfills the above needs and satisfies additional objects and advantages by providing clinically effective ampakine compositions and methods for treating attention deficit disorders. In certain aspects, new and useful methods and compositions are provided for preventing, treating and managing an attention deficit disorder (ADD). Effective treatment of ADD in mammalian subjects comprises administering to the subject one or more anti-ADD ampakines, in an amount effective to alleviate one or more symptom(s) of ADD in the subject, wherein the ampakine is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739), and wherein when the ampakine is CX717 the amount effective to alleviate one or more symptom(s) of ADD in the subject is a minimal effective daily dose greater than 400 mg and up to about 1600 mg.

In more detailed aspects, the compositions and methods of the invention are effectively used to treat attention deficit hyperactivity disorder (ADHD). These methods are effective to treat subtypes of ADHD as well including Attention Deficit Hyperactivity Disorder-predominantly hyperactive-impulsive subtype, Attention Deficit Hyperactivity Disorder-predominantly inattentive subtype, or Attention Deficit Hyperactivity Disorder-combined subtype. In related embodiments, the compositions and methods of the invention are effective to treat attention deficit disorders classified as Conduct Disorder, and Oppositional Defiant Disorder.

In related aspects, the methods and compositions of the invention effectively treat one or more symptoms of ADHD, or ADHD, in human subjects, wherein the subject presents with pediatric, adolescent, or adult hyperactivity disorder (ADHD), or a related cognitive, behavioral or neuropsychatric condition.

Within these methods and compositions, the invention provides novel medicaments and therapeutic protocols for treating ADHD and related conditions, employing ampakines that elicit potent anti-ADHD activity and other clinical effects while reducing or eliminating stimulant effects that attend the use of conventional ADHD drugs. Within exemplary embodiments of the invention, novel anti-ADHD compositions and methods are provided that employ "low impact anti-ADHD ampakines" (ampakines with strong anti-ADHD therapeutic efficacy, with substantially reduced, nominal, or no convulsant activity).

The anti-ADHD methods and compositions of the invention uniquely employ positive allosteric modulators of AMPA glutamate receptors ("ampakines"), typically "low impact" (e.g., non-convulsant) ampakines, to clinically resolve or prevent adverse symptoms of ADHD in human subjects, wherein the ampakine is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739). The clinical methods and compositions of the invention are effective in both adults and children. Important benefits of these novel ADHD drugs include minimization of stimulant activity to reducing or eliminating abuse and dependency potential and other adverse of ADHD stimulant drugs, and in the case of low impact ampakines minimizing or negating convulsant side-effects of previously-explored ampakine drugs.

Within illustrative embodiments, the invention provides compositions comprising one or more anti-ADHD ampakine(s) is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739) formulated in an effective amount and dosage form to substantially reduce or prevent one or more symptom (s) of ADHD in a human subject. Wherein when the ampakine is CX717 the amount effective to alleviate one or more symptom(s) of ADD in the subject is a minimal effective daily dose greater than 400 mg and up to about 1600 mg. In certain embodiments, the anti-ADHD cytokine will be characterized and selected as a "low impact" ampakine, having greatly reduced or no convulsant activity (e.g., as compared to convulsant activity of other, "high impact" ampakines).

In related embodiments, the invention provides clinically effective methods for treatment of attention deficit disorders, including ADHD, in mammalian subjects, including humans. The novel treatment methods of the invention include, but are not limited to, effective clinical methods for treating adult ADHD, pediatric ADHD, oppositional defiance disorder, conduct disorder.

Additionally provided herein are combinatorial compositions and coordinate treatment methods using an anti-ADHD ampakine, wherein the ampakine is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739) in a combinatorial formulation or coordinate drug administration protocol, or in a coordinate treatment regimen, in combination with one or more secondary therapeutic agents, for example a secondary anti-ADHD drug agent. Secondary therapeutic agents useful in these combinatorial formulations and coordinate therapies with anti-ADHD ampakines include anti-ADHD drug. Exemplary secondary anti-ADHD drug is a stimulant anti-ADHD drug selected from methylphenidate, methamphetamine, amphetamine, dextroamphetamine and lisdexamfetamine.

Further described herein are coordinate treatment methods and combined drug compositions, dosage forms, packages, and kits for preventing or treating ADHD and related CNS conditions.

The present invention may be understood more fully by reference to the detailed description and examples below, which are provided for illustrative purposes, understood by skilled artisans to represent non-limiting embodiments of the invention.

### BRIEF DESCRIPTION OF THE DRAWINGS

FIG. NO. 1, Panels A and B, provide standard graphical representations of electrophysiological responses (electrically evoked field excitatory postsynaptic potentials (fEPSPs)) elicited by the exemplary Anti-ADHD low impact ampakine, CX717.
Fig. NO. 2, Panels A and C, provide electrophysiological study results for an exemplary Anti-ADHD low impact ampakine CX1739. Panel B depicts anatomical placement of stimulating and recording electrodes on the brain of test subjects.
FIG. NO. 3, Panels A and B provide graphical representations of LTP data determined for the Anti-ADHD low impact ampakine CX717.
FIG. NO. 4, Panels A and B provide graphical representations of LTP data determined for the Anti-ADHD low impact ampakine CX1739.
FIG. NO. 5 graphically presents SSPA data for picrotoxin.
FIG. NO. 6 graphically presents data for assessing convulsant activity of CX717, demonstrating this exemplary compound to be a low impact ampakine with nominal or no seizure risk potential in ADHD patients at therapeutic dosage.
FIG. NO. 7 graphically presents data for assessing convulsant activity of CX614, revealing the compound is a high impact ampakine with unacceptable seizure risk potential at dosages comparable to therapeutic dosages of effective anti-ADHD ampakines.
FIG. NO. 8 is a graph providing data for spontaneous locomotor activity effects mediated by CX717 in an animal model.
FIG. NO. 9 is a graph providing data for spontaneous rearing activity effects mediated by CX717 in a distinct animal model.
FIG. NO. 10 is a graph demonstrating prolonged anti-ADHD drug efficacy of CX717 in an animal model (10 mg/kg CX717, at 1, 2, 3, and 4, hours post treatment).
FIG. NO. 11A and 11B graphically depict the effects of CX717 on amphetamine-induced locomotor activity (A), and rearing activity (B), in two different animal models.
FIG. NO. 12, panels A and B, demonstrate anti-ADHD drug efficacy of the exemplary low impact ampakine CX1739 in mouse locomotor (A), and rat rearing (B), behavioral models.
FIG. NO. 13 demonstrates anti-ADHD drug activity of the exemplary low impact ampakine CX717 in a further, novel object recognition, behavioral assay.
FIG. NO. 14 graphically depicts anti-ADHD drug activity of the exemplary low impact ampakine CX1739 in the novel object recognition behavioral model.
FIG. NO. 15, panels A and B, graphically depict anti-ADHD Drug Activity of CX717 as determined by the Radial Arm Maze behavioral assay.
FIG. NO. 16 demonstrates anti-ADHD drug activity of CX1739 identified using the Radial Arm Maze behavioral assay.
FIG. NO. 17 further demonstrates the anti-ADHD drug activity of CX 1739 using the art-accepted Five Choice Serial Reaction Task assay.
FIG. NO. 18 further demonstrates anti-ADHD drug efficacy of CX 1739 in primate Delayed Match to Sample Task behavioral assays.
FIG. NO. 19 further demonstrates the "low impact" seizure risk (nominal or no convulsant activity) of CX717. The collective data herein show this exemplary anti-ADHD ampakine is biologically active for alleviating behavioral and other target symptoms, with little or no adverse seizure risk within an indicated effective dosage range.

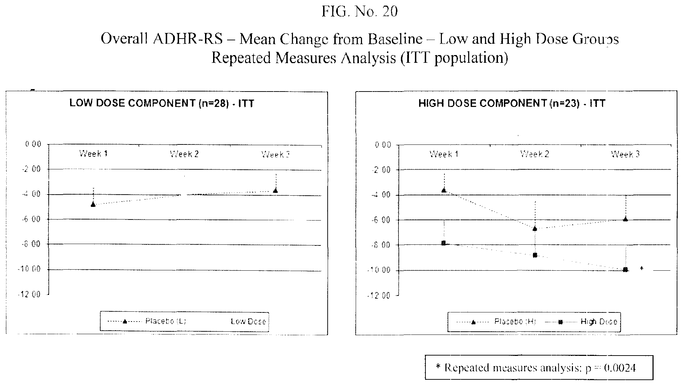

FIG. NO. 20 graphically depicts Overall ADHR-RS - Mean Change from Baseline - Low and High Dose CX717 Groups - Repeated Measures Analysis (ITT population)-Demonstrating anti-ADHD clinical efficacy of a selected low impact anti-ADHD ampakine.

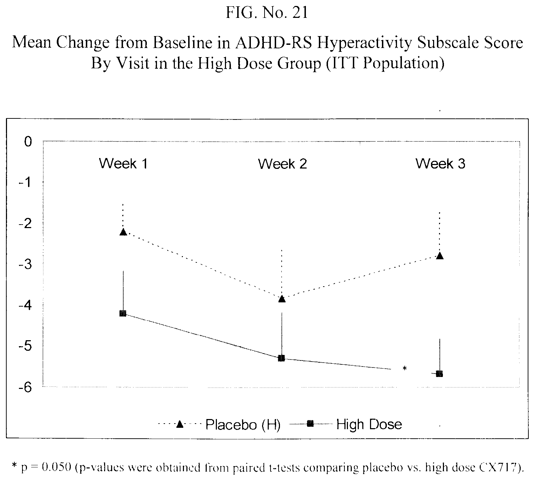

FIG. NO. 21 graphically depicts Mean Change from Baseline in ADHD-RS Hyperactivity Subscale Score by Visit in a High Dose CX717 Treatment Group (ITT Population)--Demonstrating anti-ADHD clinical efficacy and favorable safety/tolerability results for selected low impact anti-ADHD ampakine.

### DETAILED DESCRIPTION OF EXEMPLARY EMBODIMENTS

Described here are novel compositions and methods for treating attention deficit disorder (ADD) amenable to treatment using positive allosteric modulators of AMPA receptors ("ampakine"), wherein the ampakine is 1-(benzofurazan-5-ylcarbonyl)morpholine (CX717) or N-methyl-N-(tetrahydro-2H-pyran-4-yl)-[2,1,3]-benzoxadiazole-5-carboxamide (CX1739). The invention follows the surprising discovery that certain ampakines, preferably "low impact ampakines," can effectively treat identified cognitive and psychiatric CNS disorders, without unacceptable adverse side effects. In exemplary embodiments, the selected CNS disorder is a cognitive or psychiatric disorder characterized as an "attention deficit disorder".

In certain aspects the invention involves administration of an anti-ADHD effective ampakine that is uniquely effective to treat ADHD (attention deficit hyperactivity disorder), or an ADHD-related condition or symptom, in humans, with minimal adverse side effects. The anti-ADHD ampakine compositions and methods of the invention are surprisingly effective in established clinical methods, whereby they are demonstrated to treat and prevent symptoms of ADHD in adult and pediatric human patients.

In related aspects of the invention, novel compositions and methods are provided for clinical treatment and management of adult ADHD, pediatric ADHD, oppositional defiance disorder, conduct disorder.

In more detailed aspects, the methods and compostitions of the invention yield significant anti-ADHD effects without substantial adverse side effects associated with stimulant ADHD drugs (e.g., amphetamines), and with little or no attendant convulsant activity. In side-by-side clinical comparisons with stimulant ADHD drugs, including amphetamines, the anti-ADHD ampakine compounds of the invention exhibit profoundly lowered, or eliminated, stimulant effects, and likewise reduce or eliminate adverse side effects associated with the use of stimulant drugs (i.e., when tested side-by-side, at comparable anti-ADHD effective doses). In various embodiments, the anti-ADHD compositions and methods of the invention reduce or eliminate one or more stimulant drug side effects selected from: risk of drug abuse; incidence of dependency, diversion and neurotoxic effects, nervousness, headache, upset stomach, anxiety, insomnia, irritability, appetite suppression, elevated blood pressure, and motor tics, among others. For example, in side-by-side comparisons with amphetamine ADHD drugs the anti-ADHD ampakines of the invention (administered at equally effective doses for alleviating ADHD symptom(s)), yield at least a 25-35% reduction, more typically at least a 50% or 75% reduction, often as much as a 90%-95% reduction, up to complete elimination of one or more of these adverse side effects that attend treatment with ADHD stimulant drugs (e.g., amphetamines).

Examples of operable ampakines can be selected from a wide variety of known ampakine compounds (positive allosteric modulators of AMPA receptors) which, while structurally diverse as a whole, show many shared structural and functional features within classes. Both between and within known ampakine classes, useful drug candidates operable within the anti-CNS disorder can be identified, selected and proven effective according to the detailed teachings and guidance provided herein. Following these teachings, anti-CNS disorder and more discretely active anti-ADD and anti-ADHD active ampakines can be selected among positive allosteric AMPA receptor modulators from within a variety of known ampakine groups. Among the ampakine classes from which operable ampakine candidates for use can be selected include ampakines generally classified as: sulfonamide compounds and derivatives, (bis)sulfonamide compounds and derivatives, N-substituted sulfonamide compounds and derivatives; heterocyclic sulfonamide compounds and derivatives; heterocyclyl sulfonamide compounds and derivatives; alkenyl sulfonamide compounds and derivatives; cycloalkenyl sulfonamide compounds and derivatives; cyclopentyl sulfonamide compounds and derivatives; cycloalkylfluoro sulfonamide compounds and; acetylenic sulfonamide compounds and derivatives; 2-propane-sulfonamide compounds and derivatives; 2-aminobenzenesulfonamide compounds and derivatives; benzoyl piperidine and benzoyl compounds and derivatives; pyrrolidine compounds and derivatives; benzoxazine ring compounds and derivatives; acylbenzoxazine compounds and derivatives; carbonylbenzoxazine compounds and derivatives; substituted 2,3-benzodiazepin-4-one compounds and derivatives; amidophosphate; monofluoralkyl compounds and derivatives; substituted quinazoline compounds and derivatives; quainoxaline compounds and derivatives; 2-ethoxy-4'-[3-(propane-2-sulfonylamino)-thiophen-2-yl]-biphenyl-4-carboxylic and derivatives; pyrrole and pyrazole compounds and derivatives; thiadiazine compounds and derivatives; benzofurazan compounds and derivatives; benzothiazide compounds and derivatives; substituted 5-oxo-5,6,7,8-tetrahydro-4H-1-benzopyran and benzothiopyran compounds and derivatives; benzoxazepine compounds and derivatives; among known classes of compounds comprising AMPA receptor modulator compounds prospectively useful within the invention.

Anti-ADHD ampakines are selected and characterized from among various structural classes of ampakines, for example, to demonstrate low impact convulsant risk and therapeutically effective anti-ADHD activity (correlated with anti-ADHD clinical drug efficacy). Ampakines from the known class of benzofurazan ampakine compounds and derivatives (e.g., as disclosed in U.S. Pat. Nos. 6,110,935; and 6,313,115; and PCT Int'l Pub. No. WO9835950) were screened and developed to identify operable anti-ADHD drug candidates. From these investigations anti-ADHD benzofurazan compounds were identified and proven active in art-accepted assays described below, including 1-(benzofurazan-5-ylcarbonyl)-4,4-difluoropiperidine (designated "CX717"), and 4-(benzofurazan-5-ylcarbonyl).

The illustrative anti-ADHD ampakine CX717 has the chemical name 1-(benzofurazan-5-ylcarbonyl)morpholine, and corresponds to the following structure.

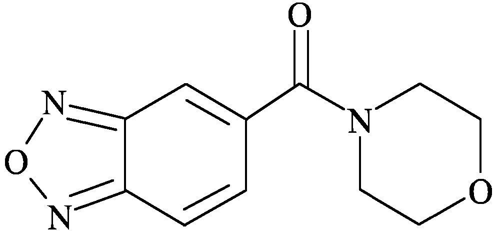

CX717 is rigorously demonstrated to be an effective anti-CNS disorder drug within the compositions and methods of the invention, and more particularly a potent anti-ADHD drug, as illustrated in the Examples below.

Within additional compositions and methods of the invention, low impact anti-ADHD ampakines are selected from another ampakine group developed by Cortex through exhaustive rational design chemistry, designated collectively as "di-substituted amide ampakines." These novel ampakines were first described by Cortex, as detailed in USSN 12/451515, US Publication No. US2010/0120764, and PCT/US/2008/00627.

Among the many di-substituted amide ampakines discovered by Cortex, three are identified here for illustrative purposes as providing useful anti-ADHD drug candidates. These are:
N-Methyl-N-tetrahydro-2H-pyran-4-yl-[2,1,3]-benzoxadiazole-5-carboxamide ("CX1739"), having the following structure. 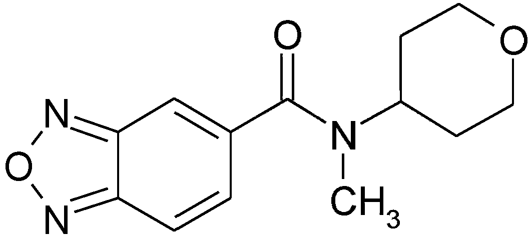
Trans-4-[(2,1,3-benzoxadiazol-5-ylcarbonyl)(methyl)amino]cyclohexyl glycinate hydrochloride (CX1942):.
   ***"CXI942"* means trans-1-[(2,1,3-Benzoxadiazol-5-ylcarbonyl)(methyl)amino]cyclohexyl glycinate hydrochloride** 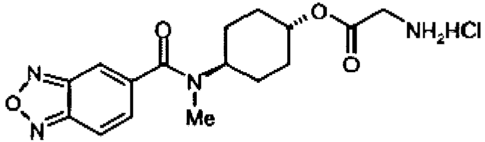
and, N-(4-trans-hydroxycyclohexyl)-N-methyl-[2,1,3]-benzoxadiazole-5-carboxamide (CX1763), having the following structure;
   ***"CX1763"* means *N*-(4-trans-Hydroxyeyclohexyl)-N-methyl-[2.1.3]-benzoxadiazole-5-carboxamide**

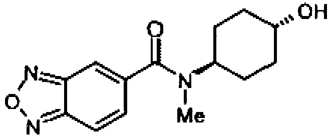

Examples of useful low impact, anti-CNS disorder, and anti-ADHD ampakines are selected and demonstrated to be active according to the teachings herein, having the exemplary ampakine structure I, below: 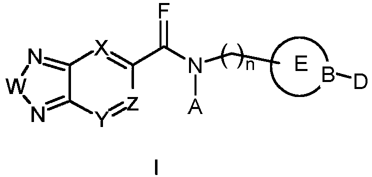 wherein:
W is oxygen, sulfur or CH=CH;
X, Y and Z are independently selected from the group consisting of -N, or -CR,
wherein:
   R is H, -Br, -Cl, -F, -CN, -NO₂, -OR¹, -SR¹, -NR¹₂, -C₁-C₆ branched or un-branched alkyl, which may be un-substituted or substituted,
   wherein:
      R¹ is H, -C₁-C₆ branched or un-branched alkyl which, may be un-substituted or substituted,
      F = O or S,
      A is H, or -C₁-C₆ branched or un-branched alkyl, which may be un-substituted or substituted, -C₂-C₆ branched or un-branched alkenyl, which may be un-substituted or substituted, -C₂-C₆ branched or un-branched alkynyl, which may be unsubstituted or substituted, -C₃-C₇ cycloalkyl which may be un-substituted or substituted, -C₃-C₇ alkylcycloalkyl which may be un-substituted or substituted, aryl or heterocycle which may be un-substituted or substituted, alkylaryl which may be un-substituted or substituted, alkylheterocycle which may be un-substituted or substituted
      n = 0, 1, 2, 3, 4, 5, or 6; 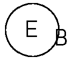 is a -C₃-C₇ cycloalkyl, which may be un-substituted or substituted, a -C₄-C₇ azacycloalkyl, which may be un-substituted or substituted, a C₇-C₁₀ bicycloalkyl which may be un-substituted or substituted, a -C₇-C₁₀ azabicycloalkyl which may be un-substituted or substituted, aryl which may be un-substituted or substituted or a heterocycle which may be un-substituted or substituted;
      B is -C=, C-R^{a}, O, N, S, C=O, S=O or SO₂;
      R^{a} is H, a halogen (preferably F), OH, O-alkyl, cyano, or a -C₁-C₆ alkyl group which is un-substituted or substituted and which optionally, forms a C₃-C₇ cycloalkyl group with D; and
      D is absent when B is O, S, S=O, C=O or SO₂, or if present, is bonded to B when B is -C=, -C-R^{a} or N, and is H, a halogen (preferably F), OR^{b}, a -C₁-C₆ branched or un-branched alkyl, which may be un-substituted or substituted and which optionally, forms a C₃-C₇ cycloalkyl group with R^{a}, a -C₂-C₆ branched or un-branched alkenyl, which may be un-substituted or substituted, a -C₂-C₆ branched or un-branched alkynyl, which may be un-substituted or substituted, a -C₃-C₇ cycloalkyl which may be un-substituted or substituted, an aryl which may be un-substituted or substituted , a heterocycle which may be un-substituted or substituted, a -C₂-C₇ carboxyalkyl which may be un-substituted or substituted, a carboxyaryl which may be unsubstituted or substituted, a carboxyheteroaryl which may be un-substituted or substituted, a -C₁-C₇ sulfonylalkyl which may be un-substituted or substituted, a sulfonylaryl which may be un-substituted or substituted or a sulfonylheteroaryl which may be un-substituted or substituted, or when B is -C-R^{a}, R^{a} and D optionally form a =N-R^{c} or a =N-OR^{c} group with B, wherein R^{c} is H or an unsubstituted or substituted C₁-C₇ alkyl group, or when B is -C-R^{a}, R^{a} and D optionally form a =N-R^{c} or a =N-OR^{c} group with B, wherein R^{c} is H or an unsubstituted or substituted C₁-C₇ alkyl group; and
      R^{b} is H, a -C₁-C₇ alkyl group which may be branched or un-branched, un-substituted or substituted or a -C₂-C₇ acyl group which may be un-substituted or substituted.

Examplesinclude compounds according to formula II below: 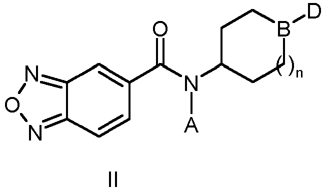 wherein:
A is -C₁-C₆ branched or un-branched alkyl, which may be un-substituted or substituted, a C₃-C₇ cycloalkyl which may be un-substituted or substituted;
n is 0, 1, 2, or 3;
B is C-R^{a}, O or C=O;
R^{a} is H, F, -OH or alkyl and
D is absent (when B is O), is H or OH when R^{a} is H or alkyl, or is F when R^{a} is F, or a pharmaceutically acceptable salt, solvate, or polymorph thereof.

Examples include compounds according to formula III below: 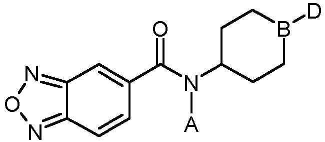 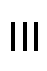 wherein:
A is a C₁-C₆ alkyl which may be substituted or un-substituted;
B is C-R^{a}, O or C=O;
R^{a} is H, F, -OH or alkyl and
D is absent (when B is O), is H or OH when R^{a} is H or alkyl, or is F when R^{a} is F, or a pharmaceutically acceptable salt, solvate, or polymorph thereof.

Examples include compounds according to formula IV below: 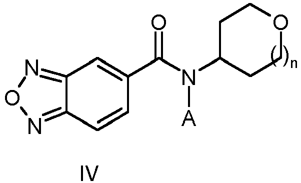 wherein:
A is a C₁-C₆ alkyl which may be substituted or un-substituted,
n is 0, 1 or 2, or a pharmaceutically acceptable salt, solvate, or polymorph thereof.

Examples include compounds according to formula V below: 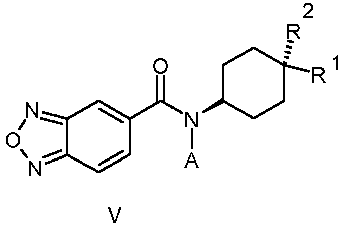 wherein:
A is a C₁-C₆ alkyl which may be substituted or un-substituted,
R¹ is H, F, or C₁-C₄ alkyl,
R² is H, F, CN, a heterocycle which may be substituted or un-substituted or OR³,
R³ is H, C₁-C₆ alkyl which may be substituted or un-substituted, or a pharmaceutically acceptable salt, solvate, or polymorph thereof.

Examples include compounds according to formula VI below: 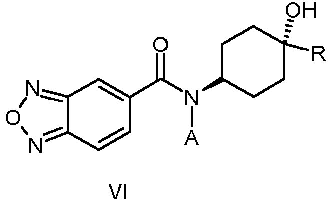 wherein:
A is a C₁-C₆ alkyl which may be substituted or un-substituted,
R is H, or C₁-C₄ alkyl, or a pharmaceutically acceptable salt, solvate, or polymorph thereof.

Examples include compounds according to formula VII below: 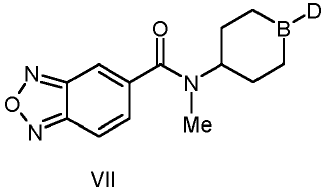 wherein:
B is C-R^{a}, O or C=O;
R^{a} is H, F, -OH or alkyl and
D is absent (when B is O), is H or OH when R^{a} is H or alkyl, or is F when R^{a} is F, or a pharmaceutically acceptable salt, solvate, or polymorph thereof.

Examples include compounds according to formula VIII below: 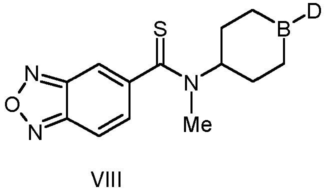 wherein:
B is C-R^{a}, O or C=O;
R^{a} is H, F, -OH or alkyl and
D is absent (when B is O), is H or OH when R^{a} is H or alkyl, or is F when R^{a} is F, or a pharmaceutically acceptable salt, solvate, or polymorph thereof.

Examples include compounds according to formula IX below: 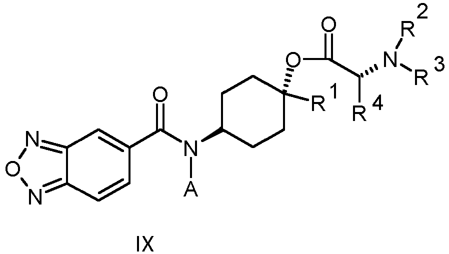 wherein:
A is a C₁-C₆ alkyl which may be substituted or un-substituted,
R¹ is H, or C₁-C₄ alkyl,
R² is H, or a C₁-C₆ alkyl which may be substituted or un-substituted,
R³ is H, or a C₁-C₆ alkyl which may be substituted or un-substituted,
R⁴ is H, or a C₁-C₆ alkyl which may be substituted or un-substituted, or a pharmaceutically acceptable salt, solvate, or polymorph thereof.

Examples of anti-CNS disorder, anti-ADD and ant-ADHD active compounds are selected from compounds of Formulas I -IX above that are already isolated and characterized, selected from: N-Cycloheptyl-N-methyl-[2,1,3]-benzoxadiazole-5-carboxamide; N-(4,4-Dimethylcyclohexyl-N-methyl-[2,1,3]-benzoxadiazole-5-carboxamide; N-Methyl-N-spiro[2.5]oct-6-yl-[2,1,3]-benzoxadiazole-5-carboxamide; N-Cyclohexyl-N-methyl-[2,1,3]-benzoxadiazole-5-carboxamide; N-Cyclopentyl-N-methyl-[2,1,3]-benzoxadiazole-5-carboxamide; N-Cyclobutyl-N-methyl-[2,1,3]-benzoxadiazole-5-carboxamide; N-Cyclohexyl-[2,1,3]-benzoxadiazole-5-carboxamide; N-Cyclopentyl-[2,1,3]-benzoxadiazole-5-carboxamide; N-Cyclobutyl-[2,1,3]-benzoxadiazole-5-carboxamide; *N-(cis-*4-Cyanocyclohexyl)-N-methyl-[2,1,3]-benzoxadiazole-5-carboxamide; *N*-(*trans*-4-Cyanocyclohexyl)-N-methyl-[2,1,3]-benzoxadiazole-5-carboxamide; *N*-Methyl-*N*-tetrahydro-2H-pyran-4-yl-[2,1,3]-benzoxadiazole-5-carboxamide (CX1739); N-D₃-Methyl-N-tetrahydro-2H-pyran-4-yl-[2,1,3]-benzoxadiazole-5-carboxamide; N-(Tetrahydro-2H-pyran-4-yl)-[2,1,3]-benzoxadiazole-5-carboxamide; N-(Tetrahydro-2H-pyran-3-yl)-[2,1,3]-benzoxadiazole-5-carboxamide; N-Methyl-N-(tetrahydro-2H-pyran-3-yl)-[2,1,3]-benzoxadiazole-5-carboxamide; *N*-Ethyl-N-tetrahydro-2H-pyran-4-yl-[2,1,3]-benzoxadiazole-5-carboxamide; N-Cyclohexyl-N-ethyl-[2,1,3]-benzoxadiazole-5-carboxamide; N-(Cyclohexylmethyl)-N-methyl-[2,1,3]-benzoxadiazole-5-carboxamide; N-Benzyl-N-methyl-[2,1,3]-benzoxadiazole-5-carboxamide; N-Methyl-N-(tetrahydrofuran-2-ylmethyl)-[2,1,3]-benzoxadiazole-5-carboxamide; *N*-Methyl-*N*-pyridin-3-yl-[2,1,3]-benzoxadiazole-5-carboxamide; N-Methyl-N-phenyl-[2,1,3]-benzoxadiazole-5-carboxamide *N*-Cyclopropyl-*N*-tetrahydro-2H-pyran-4-yl-[2,1,3]-benzoxadiazole-5-carboxamide N-Tetrahydro-2H-pyran-4-yl-N-(2,2,2-trifluoroethyl)-[2,1,3]-benzoxadiazole-5-carboxamide; tert-Butyl-4-[([2,1,3]-benzoxadiazol-5-ylcarbonyl)(methyl)amino]piperidine-1-carboxylate; N-Methyl-N-piperidin-4-yl-[2,1,3]-benzoxadiazole-5-carboxamide hydrochloride; N-Methyl-N-(1-methylpiperidin-4-yl)-[2,1,3]-benzoxadiazole-5-carboxamide; N-(1-Acetylpiperidin-4-yl)-N-methyl-[2,1,3]-benzoxadiazole-5-carboxamide; *N*-(1-Formylpiperidin-4-yl)-*N*-methyl-[2,1,3]-benzoxadiazole-5-carboxamide; *N*-Methyl-*N*-[1-(methylsulfonyl]piperidin-4-yl)-[2,1,3]-benzoxadiazole-5-carboxamide; *N-*Methyl-N-(tetrahydro-2H-pyran-4-yl)-[2,1,3]-benzothiadiazole-5-carboxamide; N-Methyl-N-(tetrahydro-2*H*-thiopyran-4-yl)-[2,1,3]-benzoxadiazole-5-carboxamide; N-Methyl-N-(1-oxidotetrahydro-2H-thiopyran-4-yl)-[2,1,3]-benzoxadiazole-5-carboxamide; N-Methyl-N-(1,1-dioxidotetrahydro-2*H*-thiopyran-4-yl)-[2,1,3]-benzoxadiazole-5-carboxamide; *N-*Methyl-N-tetrahydro-2H-pyran-4-ylquinoxaline-6-carboxamide; N-Methyl-N-(4-oxocyclohexyl)-[2,1,3]-benzoxadiazole-5-carboxamide; N-[4-(Hydroxyimino)cyclohexyl]-N-methyl-[2,1,3]-benzoxadiazole-5-carboxamide; N-[4-(Methoxyimino)cyclohexyl]-N-methyl-[2,1,3]-benzoxadiazole-5-carboxamide; N-(4,4-Difluorocyclohexyl)-N-methyl-[2,1,3]-benzoxadiazole-5-carboxamide; N-(4-fluorocyclohex-3-en-1-yl)-N-methyl-[2,1,3]-benzoxadiazole-5-carboxamide; N-(4-trans-Hydroxycyclohexyl)-[2,1,3]-benzoxadiazole-5-carboxamide; *N*-(*trans*-4-Hydroxy-4-methylcyclohexyl)-[2,1,3]-benzoxadiazole-5-carboxamide; N-(cis-4-Hydroxy-4-methylcyclohexyl)-N-methyl-[2,1,3]-benzoxadiazole-5-carboxamide; N-(trans-4-Hydroxy-4-methylcyclohexyl)-N-methyl-[2,1,3]-benzoxadiazole-5-carboxamide; N-(cis-4-Hydroxy-4-ethylcyclohexyl)-N-methyl-[2,1,3]-benzoxadiazole-5-carboxamide; N-(trans-4-Hydroxy-4-ethylcyclohexyl)-N-methyl-[2,1,3]-benzoxadiazole-5-carboxamide; *N*-(*cis*-4-Ethynyl-4-hydroxycyclohexyl)-*N*-methyl-[2,1,3]-benzoxadiazole-5-carboxamide; N-(cis-4-But-3-en-1-yl-4-hydroxycyclohexyl)-N-methyl-[2,1,3]-benzoxadiazole-5-carboxamide; N-(trans-4-But-3-en-1-yl-4-hydroxycyclohexyl)-N-methyl-[2,1,3]-benzoxadiazole-5-carboxamide; *N*-(4-trans-Hydroxycyclohexyl)-*N*-methyl-[2,1,3]-benzoxadiazole-5-carboxamide (CX1763); N-(4-trans-Hydroxycyclohexyl)-N-Ds-methyl-[2,1,3]-benzoxadiazole-5-carboxamide; N-(trans-4-Methoxycyclohexyl)-N-methyl-[2,1,3]-benzoxadiazole-5-carboxamide; N-(trans-4-Methoxycyclohexyl)-N-methyl-[2,1,3]-benzoxadiazole-5-carbothioamide; N-(4-cis-Hydroxycyclohexyl)-N-methyl-[2,1,3]-benzoxadiazole-5-carboxamide; *N-*Methyl-*N*-[*trans*-4-(2*H*-tetrazol-2-yl)cyclohexyl]-[2,1,3]-benzoxadiazole-5-carboxamide; *N*-(trans-4-Azidocyclohexyl)-*N*-methyl-[2,1,3]-benzoxadiazole-5-carboxamide; N-(trans-4-Aminocyclohexyl)-N-methyl-[2,1,3]-benzoxadiazole-5-carboxamide; N-(cis-3-Hydroxycyclohexyl)-N-methyl-[2,1,3]-benzoxadiazole-5-carboxamide; N-(trans-3-Hydroxycyclohexyl)-N-methyl-[2,1,3]-benzoxadiazole-5-carboxamide; N-Methyl-N-(3-oxocyclohexyl)-[2,1,3]-benzoxadiazole-5-carboxamide; N-Methyl-N-(3,3-difluorocyclohexyl)-[2,1,3]-benzoxadiazole-5-carboxamide; N-(2-Hydroxycyclohexyl)-N-methyl-[2,1,3]-benzoxadiazole-5-carboxamide; N-Methyl-N-(2-oxocyclohexyl)-[2,1,3]-benzoxadiazole-5-carboxamide; N-Methyl-N-(2,2-difluorocyclohexyl)-[2,1,3]-benzoxadiazole-5-carboxamide; N-(2-Hydroxytetrahydro-2H-pyran-4-yl)-[2,1,3]-benzoxadiazole-5-carboxamide; N-(2-oxotetrahydro-2H-pyran-4-yl)-[2,1,3]-benzoxadiazole-5-carboxamide; N-Methyl-N-(2-oxotetrahydro-2H-pyran-4-yl)-[2,1,3]-benzoxadiazole-5-carboxamide; N-(2-Hydroxytetrahydro-2H-pyran-4-yl)-N-methyl-[2,1,3]-benzoxadiazole-5-carboxamide; trans-4-[(2,1,3-Benzoxadiazol-5-ylcarbonyl)(methyl)amino]cyclohexyl N,N-dimethyl glycinate hydrochloride; trans-4-[(2,1,3-Benzoxadiazol-5-ylcarbonyl)(methyl)amino]cyclohexyl L-alaninate hydrochloride; N- (R)-Tetrahydrofuran-3-yl-[2,1,3]-benzoxadiazole-5-carboxamide; N-Methyl-N-(R)-tetrahydrofuran-3-yl-[2,1,3]-benzoxadiazole-5-carboxamide; trans-4-[(2,1,3-Benzoxadiazol-5-ylcarbonyl)(methyl)amino]cyclohexyl glycinate hydrochloride; N-2-(4-Morpholinyl)ethyl-[2,1,3]-benzoxadiazole-5-carboxamide; N-Methyl-N-2-(4-morpholinyl)ethyl-[2,1,3]-benzoxadiazole-5-carboxamide hydrochloride; N-Methyl-N-tetrahydro-2H-pyran-4-yl-[2,1,3]-benzoxadiazole-5-carbothioamide (CX1739); trans-4-[(2,1,3-Benzoxadiazol-5-ylcarbonyl)(methyl)amino]cyclohexyl L-valinate hydrochloride; *trans*-4-[(2,1,3-Benzoxadiazol-5-ylcarbonyl)(methyl)amino]-1-methylcyclohexyl N,N-dimethyl glycinate hydrochloride; N-Methyl-N-tetrahydro-2H-pyran-4-ylmethyl-[2,1,3]-benzoxadiazole-5-carboxamide; and trans-4-[(2,1,3-Benzoxadiazol-5-ylcarbonyl)(methyl)amino]-1-methylcyclohexyl glycinate hydrochloride (CX1942).

The synthesis of compounds for use within the invention is described generally elsewhere in the references provided herein. Briefly, for di-substituted amide compounds above, production of N-methylamines is carried out using standard protocols, for example, amines are dissolved in a suitable organic solvent, for example dichloromethane, a base (e.g. NEt₃ or NaHCO₃ in water) is added and then a solution of benzyloxycarbonyl chloride (Cbz-Cl) in an organic solvent e.g. dichloromethane is added, which results in the formation of the benzyl carbamates. The carbamates are then reduced with, for example, lithium aluminum hydride (LiAlH₄) in a suitable organic solvent for example tetrahydrofuran (THF) to yield amines. The amines may be alternatively prepared by reductive amination of ketones 3 in the presence of an amine (ANH₂), using standard conditions, for example Pd/C in an appropriate solvent for example ethanol. Acid chloride may be synthesized starting with 4-amino-3-nitrobenzoic acid 5, by firstly oxidizing using sodium hypochlorite in ethanol in the presence of potassium hydroxide to give intermediate and then reducing with triethyl phosphate (P(OEt)₃) in a suitable solvent, for example ethanol, to give benzofurazan carboxylic acid. The carboxylic acid can be transformed to the acid chloride refluxing with thionyl chloride in toluene. The benzofurazan carboxylic acid can be transformed into amides using amines, using standard amide coupling conditions for example 1-ethyl-3-(3-dimethylaminopropyl)carbodiimide (EDCI), O-benzotriazole-N,N,N',N'-tetramethyl-uronium-hexafluoro-phosphate (HBTU), N-hydroxybenzotriazole (HOBT), dimethylaminopyridine (DMAP) and triethyalamine in a suitable solvent e.g. dichloromethane. Alternatively, acid chloride can be transformed into amides using standard coupling conditions with amines in the presence of a base for example triethylamine in dicloromethane as solvent or aqueous sodium hydrogen carbonate in water and dichloromethane. The benzothiadiazole amides can be prepared from commercially available benzothiadiazole acid chloride using standard coupling conditions with amines in the presence of a base for example triethylamine in dicholoromethane as solvent, or aqueous sodium hydrogen carbonate in water and dichloromethane. Quinoxaline-6-carboxylic acid chloride is prepared by condensation of commercially available 3,4-diaminobenzoic acid with glyoxal followed by refluxing with thionyl chloride and a catalytic amount of DMF in toluene using standard procedures, with conversion to amides by coupling reaction with amines using standard procedures. Alternatively, amides can be prepared from other amides by deprotonation with a suitable base for example sodium hydride in a solvent e.g. N,N-dimethylformamide (DMF) followed by treatment with an alkylating agent (RX) to yield the desired product. For example, thioamides can be prepared from amides by reacting 10 with phosphorous pentoxide in a suitable solvent e.g. toluene. Adaptation of these and other chemical synthetic methods and intermediates to produce additional active ampakine compounds within the invention is within the skill and knowledge of the ordinary artisan.

Within additional compositions and methods of the invention, low impact anti-ADHD ampakines are selected from yet additional ampakine groups developed by Cortex or others including Cortex' "bicyclic amide ampakines." Among the many bicyclic amide ampakines identified by Cortex for evaluation to determine usefulness within anti-CNS disorder, and more specifically anti-ADD and anti-ADHD efficacy, are the following exemplary species:
8-Azabicyclo[3.2.1]oct-8-yl([2,1,3]-benzoxadiazol-5-yl)methanone; 8-([2,1,3]-Benzoxadiazol-5-ylcarbonyl)-8-azabicyclo[3.2.1]octan-3-one; [2,1,3]-Benzoxadiazol-5-yl(3,3-difluoro-8-azabicyclo[3.2.1]oct-8-yl)methanone; endo-[2,1,3]-Benzoxadiazol-5-yl(3-hydroxy-8-azabicyclo[3.2.1]oct-8-yl)methanone; exo-[2,1,3]-Benzoxadiazol-5-yl(3-hydroxy-8-azabicyclo[3.2.1]oct-8-yl)methanone; 2-Azabicyclo[2.2.1]hept-2-yl([2,1,3-benzoxadiazol-5-yl)methanone; 1-Azabicyclo[2.2.1]hept-1-yl([2,1,3]-benzoxadiazol-5-yl)methanone; 2-Azabicyclo[2.2.2]oct-2-yl([2,1,3]-benzoxadiazol-5-yl)methanone; [2,1,3]-Benzoxadiazol-5-yl(5,6-dichloro-2-azabicyclo[2.2.1]hept-2-yl)methanone.

Additional bicyclic amide ampakines for prospective use within the anti-CNS disorder, anti-ADD and anti-ADHD methods and compositions of the invention include, but are not limited to, the following exemplary species:
[2,1,3]-Benzoxadiazol-5-yl(3-fluoro-8-azabicyclo[3.2.1]oct-2-en-8-yl)methanone; 2-Azabicyclo[2.2.1]hept-5-en-2-yl([2,1,3]-benzoxadiazol-5-yl)methanone; R-2-Azabicyclo[2.2.1]hept-5-en-2-yl([2,1,3]-benzoxadiazol-5-yl)methanone; S-2-Azabicyclo[2.2.1]hept-5-en-2-yl([2,1,3]-benzoxadiazol-5-yl)methanone; and [2,1,3]-Benzoxadiazol-5-yl(2-oxa-5azabicyclo[2.2.1]hept-5-yl)methanone.

Additional ampakine compounds for use within the invention will be selected according to the teachings herein, using known AMPA receptor modulator compounds, reagents, preparative methods and other tools as disclosed in the following publications: PCT Int'l Pub. No. WO 94/02475 and related U.S. Pat. Nos. 5,773,434, 5,488,049, 5,650,409, 5,736,543, 5,747,492, 5,773,434, 5,891,876, 6,030,968, 6,274,600, 6,329,368, 6,943,159, and 7,026,475; U.S. Pat. Pub. No. 20020055508; U.S. Pat. Nos. 6,174,922, 6,303,816, 6,358,981, 6,362,230, 6,500,865, 6,515,026, and 6,552,086; PCT Int'l Pub. Nos. WO 0190057, WO 0190056, WO 0168592, WO 0196289, WO 02098846, WO 0006157, WO 9833496, WO 0006083, WO 0006148, WO 0006149, WO 9943285, and WO 9833496; WO 0194306; U.S. Pat. No. 6,525,099 and PCT Int'l Pub. No. WO 0006537; U.S. Pat. No. 6,355,655 and PCT Int'l Pub. Nos. WO0214294, WO0214275, and WO0006159; U.S. Pat. No. 6,358,982 and PCT Int'l Pub. No. WO0006158; U.S. Pat. No. 6,387,954 and PCT Int'I Pub. No. WO0006539; PCT Int'l Pub. No. WO02098847; U.S. Pat. No. 6,639,107 and PCT Int'I Pub. No. WO0142203; PCT Int'l Pub. No. WO0232858; PCT Int'l Pub. No. WO0218329; U.S. Pat. No. 6,596,716 and PCT Int'l Pub. Nos: WO2006087169, WO2006015827, WO2006015828, WO2006015829, WO2007090840, and WO2007090841; WO02089734; U.S. Pat. Nos. 5,650,409, 5,747,492, 5,783,587, 5,852,008, and 6,274,600; U.S. Pat. Nos. 5,736,543, 5,962,447, 5,985,871, and PCT Int'l Pub. Nos. WO 9736907 and WO9933469; U.S. Pat. No. 6,124,278, and PCT Int'l Pub. No. WO 9951240; PCT Int'l Pub. No. WO03045315; U.S. Pat. No. 5,891,871; U.S. Pat. Nos. 6,110,935 and 6,313,115, and PCT Int'l Pub. No. WO9835950; PCT Int'I Pub. No. WO 9812185; PCT Int'l Pub. No. WO0075123; U.S. Pat. No. 6,521,605 and PCT Int'I Pub. No. WO0006176; PCT Int'l Pub. No. WO 0066546; PCT Int'l Pub. No. WO 9944612; PCT Int'l Pub. No. WO2007060144; U.S. Pat. Pub. No. 20060276532; U.S. Pat. Pub. No. 20070066573; U.S. Pat. Pub. No. 20070004709; U.S. Pat. Pub. No. 20040171605; PCT Int'I Pub. Nos. WO 9942456, WO 0006156, and WO 0157045, and U.S. Pat. No. 6,617,351.

Additional description and background pertaining also to specific positive allosteric AMPA receptor modulators, their preparation, use and selection within the compositions and methods of the invention, is provided in the following references: For benzofurazan compounds-PCT patent application PCT/US98/02713, United States patent application Serial No. 08/800,108, now United States Patent 6,110,935, United States patent application Serial No. 09/355,139, now United States Patent 6,313,115, United States patent application, Serial No. 09/834,349; United States patent application, Serial No. 09/845,128, now United States Patent 6,730,677; For di-substituted amide ampakines-PCT patent application PCT/US2008/006271, United States patent application Serial No. 12/451,515, now United States Patent 8,013,003, United States patent application, Serial No. 13/226,146, now issued United States Patent 8,404,682, and United States patent application, Serial No. 13/755,210, now issued United States Patent 8,642,633; For bicyclic amide ampakines-PCT patent application PCT/US2008/009508, United States Provisional patent application, Serial No. 60/964,362; United States patent application, Serial No. 12/657,908, now United States Patent 8,119,632, United States patent application, Serial No. 12/733,073, now United States Patent 8,263,591, United States patent application, Serial No. 13/348,171, now United States Patent 8,507,482, United States patent application, Serial No. 13/557,681, United States patent application, Serial No. 12/657,924, now United States Patent 8,168,632, PCT patent application PCT/US2010/000255, and United States Provisional patent application, Serial No. 61/206,642; For bicyclic amide ampakines-PCT patent application PCT/US2010/000254, and United States Provisional patent application, Serial No. 61/206,642; For 3-Substituted-[1,2,3]Benzotriazinone ampakines-PCT Patent application PCT/US2007/026415, United States Provisional patent application, Serial No. 60/878,626, United States patent application, Serial No. 12/448,770, PCT patent application PCT/US2007/026416, United States Provisional application, Serial No. 60/878,503, United States Provisional patent application, Serial No. 60/921,433, and United States patent application, Serial No. 12,448,784, now United States Patent 8,173,644; For 3-substituted 1,2,3-triazin-4-one and 3-substituted 1,3-pyrimidinone ampakines-PCT patent application PCT/US2008/010877, United States Provisional patent application, Serial No.60/994,548, and United States patent application, Serial No. 12/733,822; For benzoxazine ampakines-PCT patent application PCT/US98/27027, and United States patent application, Serial No. 08/998,300, now United States Patent 5,985,871; for Acylbenzoxazines ampakines- PCT patent application, Serial No. PCT/US99/07325, and United States patent application 09/054,916, now United States Patent 6,124,278; for Benzoyl Piperidine/Pyrrolidine ampakines PCT patent application PCT/US96/07607, and United States patent application Serial No. 08/458,967, filed 2 June 1995, now United States Patent 5,650,409; For benzoxazine ampakines-PCT patent application, Serial No. PCT/US97/05184, United States patent application, Serial No. 08/624,335, now United States Patent 5,736,543, PCT patent application PCT/US93/06916, and United States patent application, Serial No. 07/919,512, now United States Patent 5,962,447; and for carbonylbenzoxazine ampakines-PCT patent application PCT/US02/37646, United States Provisional patent application, Serial No. 60/333,334, and United States patent application Serial No. 10/495,049, now United States Patent 7,799,913.

Each of the foregoing classes and distinct structural groups of ampakine compounds disclosed in the above references are suitable for evaluation to determine operability within the methods and compositions of the invention. Persons of ordinary skill in the art will recognize that these various compound groups, while being structurally diverse, share common functional characteristics of positive allosteric AMPA receptor modulation, as described here, and that because of these common functional characteristics, the compounds can be evaluated and determined for their operability according to the inventive discoveries and teachings provided herein. According to the Examples and other guidance provided here, anti-ADHD ampakines, for example, can be anti-CNS disorder and anti-ADD drug candidates can be selected and demonstrated, for example, to be therapeutically anti-ADHD effective for beneficial, clinical use. Following the teachings of this invention, many additional active drug candidates, for example drugs therapeutically effective as anti-ADHD compounds, can be selected from different classes and subgroups of ampakines described here, without undue experimentation.

The anti-CNS disorder ampakines of the invention, when administered to subjects, will yield a reduction in one or more target symptom(s) associated with the selected CNS disorder by at least 2%, 5%, 10%, 20%, 30%, 50% or greater, up to a 75-90%, or 95% or greater, compared to placebo-treated or other suitable control subjects. Comparable levels of efficacy are contemplated for the entire range of disorders described herein, including all contemplated CNS, cognitive, behavioral, neurological and psychiatric disorders, and related conditions and symptoms, including for effective treatment of ADD, ADHD, or one or more attendant symptoms thereof. These values for efficacy may be determined by comparing accepted therapeutic indices or clinical values for particular test and control individuals over a course of treatment/study, or more typically by comparing accepted therapeutic indices or clinical values between test and control groups of individuals using standard human clinical trial design and implementation.

As used herein, the terms "prevention" and "preventing," when referring to a CNS disorder or symptom, refers to a reduction in the risk or likelihood that a subject, e.g., a human patient, will develop the disorder, symptom, condition, or indicator after treatment according to the invention, or a reduction in the risk or likelihood that the subject will exhibit a recurrence or relapse of said disorder, symptom, condition, or indicator once treated according to the invention and restored toward a normal state (e.g., placed in remission from a targeted disorder). As used herein, the terms "treatment" or "treating," when referring to the targeted disorder, refers to inhibiting or reducing the progression, nature, or severity of the subject condition or delaying the onset of the condition.

In accordance with the invention, compounds disclosed herein, optionally formulated with additional ingredients in a pharmaceutically acceptable composition, are administered to mammalian subjects, for example a human patient, to treat or prevent one or more symptom(s) of a CNS disorder alleviated by positive allosteric AMPA receptor modulation employing a selected ampakine as described herein. In certain embodiments, "treatment" or "treating" refers to amelioration of one or more symptom(s) of a disorder, whereby the symptom(s) is/are alleviated by an anti-CNS disorder (e.g., anti-ADHD) ampakine drug. In other embodiments, "treatment" or "treating" refers to an amelioration of at least one measurable physical parameter associated with a disorder. In yet another embodiment, "treatment" or "treating" refers to inhibiting or reducing the progression or severity of a CNS disorder (e.g., one or more symptom(s) of ADHD) alleviated by selected ampakine administration, as discerned for example based on physical, physiological, behavioral and/or psychological parameters. In additional embodiments, "treatment" or "treating" refers to delaying the onset of a disorder (or one or more symptom(s) thereof) alleviated by administration of an ampakine within the subject compositions and methods of the invention.

An "effective amount," "therapeutic amount," "therapeutically effective amount," or "effective dose" of an anti-CNS disorder or anti-ADHD ampakine means an effective amount or dose of the active compound as described herein sufficient to elicit a desired pharmacological or therapeutic effect in a mammalian subject, typically a human patient. In the case of therapeutic agents for ADHD, for example, these terms most often correlate with a significant reduction in an occurrence, frequency, or severity of one or more recognized symptom(s) of ADHD, including any combination of neurological, functional, behavioral, and/or psychological symptoms with ADHD.

Briefly elaborating these therapeutic indices for the example of attention deficit CNS disorders, ADHD is characterized by symptoms of: difficulty staying focused and paying attention, difficulty controlling behavior, impulsivity, disorganization, and hyperactivity (over-activity). ADHD is diagnosed in both children and adults based on well known and widely accepted diagnostic/symptom logical criteria, for example as described in the Diagnostic and Statistical Manual of Mental Disorders, Fourth Edition (2000), Text Revision (DSM-IV-TR), American Psychiatric Association, Washington, D.C. The DSM-IV-TR criteria describe three subtypes of ADHD: Attention Deficit Hyperactivity Disorder-predominantly hyperactive-impulsive subtype; Attention Deficit Hyperactivity Disorder-predominantly inattentive subtype (also referred as Attention Deficit Disorder or ADD); and Attention Deficit Hyperactivity Disorder-combined subtype. In the predominantly inattentive type, a person may present with six or more of the following disruptive and age-inappropriate symptoms: difficulty paying attention to details, difficulty keeping attention on tasks, difficulty following instructions, difficulty organizing activities, difficulty following conversations, being easily distracted, and forgetful of daily routines. In the predominantly hyperactive-impulsive type, a person can have six or more of the following disruptive and age-inappropriate symptoms: fidgeting often, inappropriate running about, trouble playing or enjoying leisure activities quietly, excessive talking, blurting out answers, trouble waiting turn, and interrupting others. In the combined type, both inattentive and hyperactive-impulsive behaviors can be present. Anti-ADHD ampakines employed within the compositions and methods of the invention will be effective to significantly alleviate one or more of these symptoms, or effectively reduce a "score" or "scale" comprising a suite of observed symptoms, on a therapeutic basis (e.g., percent improvement) as described.

Patients treated with the anti-ADHD drugs, or coordinate drug treatments, of the invention, will typically exhibit a reduction of symptoms, or reduced occurrence (prevention) of symptoms, marked by at least a 10%, 25%, 30-40%, 50%, 75%, 90-95%, up to 95% or higher, reduction of scores (indicating ADHD severity) on one or more ADHD clinical assessment scales selected from the Adult ADHD Rating Scale (DuPaul, 1998) with prompts (ADHD-RS) (Adler & Cohen, 2004). , Clinical Global Impressions Scale - Improvement (CGI-I) (Guy, 1976), Adult ADHD Self-Rating Scale (ASRS) (Kessler, 2005), Hamilton Anxiety Scale (HAM-A) (Hamilotn, 1959), Hamilton Depression Scale (HAM-D) (Hamilton, 1960), sleep assessment scales (Pittsburg Sleep Quality Inventory [PSQI] (Buysse, 1989), Epworth Sleepiness Scale [ESS] (Johns, 1991), and neuropsychometric tests (Stroop Color and Word Test (Gamble & Kellner, 1968), Comprehensive Trail-Making Test [CTMT] (Reynolds, 2002), Forward and Backward Digit Span (Jensen & Figueroa, 1975), and the Conners' Continuous Performance Test [CPT] (Conners, 2000).

Continuing with the example of ADHD, anti-ADHD ampakines selected for use within the invention are effective in treating all subtypes of ADHD, both in adult and pediatric subjects. This efficacy encompasses all types of attention deficit hyperactivity disorder, as well as related conditions designated as Conduct Disorder; Oppositional Defiant Disorder.

Efficacy of the treatment compositions and methods of the invention, including coordinate treatment methods and combined drug compositions, will often be determined by use of conventional patient surveys or clinical scales to measure clinical indices of disorders in subjects. The methods and compositions of the invention will yield a reduction in one or more scores or selected values generated from such surveys or scales completed by test subjects (indicating for example an incidence or severity of a selected disorder), by at least 5%, 10%, 20%, 30%, 50% or greater, up to a 75-90%, or 95% compared to correlative scores or values observed for control subjects treated with placebo or other suitable control treatment. In at risk populations, the methods and compositions of the invention will yield a stable or minimally variable change in one or more scores or selected values generated from such surveys or scales completed by test subjects. More detailed data regarding efficacy of the methods and compositions of the invention can be determined using alternative clinical trial designs.

Useful patient surveys and clinical scales for comparative measurement of clinical indices of CNS disorders in subjects treated using the methods and compositions of the invention can include any of a variety of widely used and well known surveys and clinical scales. Several surveys are available for ADHD, including assessments for adults, children, those rated by clinical investigators, and those rated by subjects treated. Among these useful tools are the Wender Utah Rating Scale (WURS) (Ward et al., 1993); Adult Rating Scale (ARS) (Weyandt et al., 1995); Current Symptoms Scale (CSS) (Barkley and Murphy, 1998); Conners Adult ADHD Rating Scale (CAARS) (Conners et al., 1999); Adult Problems Questionnaire (APQ) (De Quiros and Kinsbourne, 2001); Young Adult Rating Scale (YARS) (Du Paul et al., 2001); Assessment of Hyperactivity and Attention (AHA) (Mehringer et al., 2002); Attention Deficit Scales for Adults (ADSA) (Triolo and Murphy, 1996); ADHD Rating Scale (ADHD-RS) (Du Paul *et al.,* 1998); Brown Attention Deficit Disorder Series (BADDS) (Brown, 1996); Symptom Inventory (SI) (McCann and Roy-Byrne, 2004); Young Adult Questionnaire (YAQ) (Young, 2004); Adult Self Report Scale (ASRS) (Adler et al., 2006), and the Caterino Scale (Caterino et al., 2009).

The methods and compositions of the invention will yield a reduction in one or more scores or values generated from these and/or other clinical surveys or scales (using any single scale or survey, or any combination of one or more of the surveys such as those described above) by at least 10%, 20%, 30%, 50% or greater, up to a 75-90%, or 95% compared to correlative scores or values observed for control subjects treated with placebo or other suitable control treatment. In prophylactic treatment, the methods and compositions of the invention will yield a stabilization or diminished change in the scores or values generated from these clinical surveys.

Within additional aspects of the invention, combinatorial formulations and coordinate treatment methods are provided that employ an effective amount of an ampakine, for example an anti-ADHD ampakine, and one or more "secondary" therapeutic agent(s)". The secondary agent is co-formulated or coordinately administered with the ampakine compound, to yield a combined formulation or coordinate treatment method that is effective to treat or prevent a targeted CNS disorder in a mammalian subject. Exemplary combinatorial formulations and coordinate treatment methods for ADHD comprise an anti-ADHD ampakine combined with one or more adjunctive treatment agents for treating ADHD. Alternatively the secondary therapeutic agent can possess distinct activity for treating a co-morbid condition, for example an anxiolytic or antidepressant to treat anxiety or depression co-occurring in an ADHD patient.

In related embodiments any ampakine disclosed herein can be employed in combination therapy with one or more secondary therapeutic agents to treat ADHD or another CNS disorder. In illustrative embodiments, an anti-ADHD ampakine can be administered concurrently or sequentially with the secondary therapeutic agent, and the secondary agent will act additively, synergistically or distinctly to treat or prevent the same, or different, disorder or symptom(s) for which the anti-ADHD ampakine is administered. The anti-ADHD ampakine and the secondary therapeutic may be combined in a single formulation, or separately administered at the same or different time. Administration of the two drugs separately may be done simultaneously or sequentially in either order, and a therapeutic interval may include time(s) when only one or both (or all) active therapeutic agents individually and/or collectively exert their therapeutic effect. A distinguishing aspect of all such coordinate treatment methods is that the selected ampakine exerts at least some detectable therapeutic activity toward alleviating or preventing the targeted disorder or symptom(s), as described herein, and elicits a favorable clinical response, which may or may not be in conjunction with a separate or enhanced clinical response mediated by the secondary therapeutic agent. In other illustrative embodiments, coordinate administration of the an anti-ADHD ampakine with a secondary therapeutic agent will typically yield a greater therapeutic response compared to clinical responses observed following administration of either the ampakine or the secondary agent alone at the same dosage, with reduced side effects. For example, coordinate treatment using a stimulant anti-ADHD drug with an anti-ADHD ampakine yields full therapeutic effects with an individually sub therapeutic dose of the stimulant, avoiding or lessening stimulant-associated side effects (i.e., compared to the side effects observed using an equal therapeutic dose of the stimulant alone).

In coordinate therapies of the invention where the secondary therapeutic agent is a secondary anti-ADHD drug, this adjunctive treatment agent will often be a conventional, stimulant anti-ADHD drug. Exemplary drugs of this type include, but are not limited to, methylphenidate, methamphetamine, amphetamine, dextroamphetamine and lisdexamfetamine. In related embodiments the stimulant anti-ADHD drug may be administered at a sub-therapeutic dose, either in a combinatorial formulation or coordinate administration protocol with the anti-ADHD ampakine, yielding a therapeutically effective combined formulation or coordinate treatment protocol. The surprising combinatorial efficacy of the anti-ADHD ampakine combined with a normally (i.e., administered solo) sub therapeutic dose of the stimulant drug, provides an important advantage of eliminating or substantially reducing the stimulant impact of the treatment, along with other adverse side effects that attend solo administration of the stimulant anti-ADHD drug alone (in a full, individually therapeutic dose). These relative dosage regimes employed here will vary, for example according to well-known clinical and patient-specific parameters, while the terms "therapeutic dose" and "sub therapeutic dose" have ordinary and clear meaning to persons skilled in the art (and are here applicable to any one or combination of symptoms associated with the targeted CNS condition, e.g., any one or more recognized ADHD symptom(s)).

In other combinatorial formulations and coordinate treatment methods of the invention, the secondary anti-ADHD drug may be a non-stimulant anti-ADHD drug. For example, adjunctive treatment employing an anti-ADHD ampakine with atomoxetine, clonidine, guanfacine and/or any other known ADHD drug is contemplated within the invention. This yields enhanced anti-ADHD therapeutic results and/or reduced side effects (for example by lowering dosage for one or both of the anti-ADHD ampakine, and/or secondary drug to individually sub therapeutic level(s) obviating or reducing side effects associated with full therapeutic dosage of one or both drugs).

In additional embodiments, secondary therapeutic agent is a drug selected to treat a co-morbid condition in a patient being treated with the ampakine. Secondary treatment targets that may occur as co-morbid conditions with ADHD, for example, include anxiety, depression, seizures and many other conditions. Thus in various aspects the invention employs a secondary therapeutic agent selected from an antipsychotic, antidepressant, anti-convulsant, mood-stabilizer, anxiolytic, benzodiazepine, calcium channel blocker, or anti-inflammatory drug, or any combination thereof.

Useful antipsychotic drug within these embodiments include conventional antipsychotics, as well as atypical antipsychotics, for example aripiprazole, ziprasidone, risperidone, quetiepine, or olanzapine.

Useful anti-convulsant drugs for combinatorial use with ampakines according to the invention can include, for example, acetazolamide, carbamazepine, clonazepam, ethosuximide, gabapentin, lacosamide, lamotrigine, levetiracetam, nitrazepam, oxcarbazepine, piracetam, phenobarbital, phenytoin, pregabalin, primidone, rufinamide, sodium valproate, topiramate, vigabatrin and zonisamide, among many others.

Useful anxiolytic agents for combinatorial use with ampakines include barbiturates, benzodiazepines and others. Exemplary benzodiazepines include alprazolam, chlordiazepoxide, clobazepam, clonazepam, clorazepate, diazepam, estazolam, and flurazepam

In other combinatorial formulations and coordinate treatment methods provided herein, the secondary therapeutic agent is an anti-depressant drug, for example to treat patients with ADHD and co-morbid depression. The secondary condition in this circumstance can include depression, atypical depression, or treatment-resistant depression, among other related conditions. In these exemplary aspects, an anti-ADHD ampakine is coordinately administered with one or more antidepressants as the secondary therapeutic agent. Antidepressants selected for this purpose can include any known antidepressant drug, including tri-cyclic antidepressants (TCAs), specific monoamine reuptake inhibitors, selective serotonin reuptake inhibitors (SSRIs), selective norepinephrine or noradrenaline reuptake inhibitors, selective dopamine reuptake inhibitors, norepinephrine-dopamine reuptake inhibitors, serotonin-norepinephrine reuptake inhibitors, multiple monoamine reuptake inhibitors, monoamine oxidase inhibitors, and atypical antidepressants.

Exemplary tri-cyclic antidepressants (TCAs) that can be co-formulated or coadministered with anti-ADHD ampakines, for example, include amitriptyline, imipramine, clomipramine, or desipramine. Useful SSRIs include, but are not limited to, escitalopram, fluoxetine, fluvoxamine, sertraline, citalopram, vilazodone, and paroxetine. Useful selective norepinephrine or noradrenaline reuptake inhibitors including, but are not limited to, tertiary amine tricyclics such as amitriptyline, clomipramine, doxepin, imipramine, (+)-trimipramine, and secondary amine tricyclics including amoxapine, desipramine, maprotiline, nortriptyline, and protriptyline. Useful multiple monoamine reuptake inhibitors, e.g., that inhibit both serotonin and norepinephrine reuptake (SNRIs), include, but are not limited to, venlafaxine, duloxetine, milnacipran, and sibutramine. Useful multiple reuptake inhibitors for both norepinephrine and dopamine include, but are not limited to bupropion, amineptine, prolintane, dexmethylphenidate or pipradrol. Useful inhibitors of both serotonin and dopamine include monoamine oxidase inhibitors (MAOIs); and indeterminate (atypical) antidepressants.

In additional combinatorial formulations and coordinate treatment methods provided herein, the secondary therapeutic agent is an anti-addictive-disorder or anti-substance abuse agent. These agents may be employed, for example, in adult patients with ADHD who present with prior or concurrent drug addiction issues. Examples of useful anti-addictive-disorder agents for coordinate treatment of these subjects include, but are not limited to, tricyclic antidepressants; glutamate antagonists, such as ketamine HCl, dextromethorphan, dextrorphan tartrate and dizocilpine (MK801); degrading enzymes, such as anesthetics and aspartate antagonists; GABA agonists, such as baclofen and muscimol HBr; reuptake blockers; degrading enzyme blockers; glutamate agonists, such as D-cycloserine, carboxyphenylglycine, L-glutamic acid, and cis-piperidine-2,3-dicarboxylic acid; aspartate agonists; GABA antagonists such as gabazine (SR-95531), saclofen, bicuculline, picrotoxin, and (+) apomorphine HCl; and dopamine antagonists, such as spiperone HCl, haloperidol, and (-) sulpiride; anti-alcohol agents including, but not limited to, disulfiram and naltrexone; anti-nicotine agents including but not limited to, clonidine; anti-opiate agents including, but not limited to, methadone, clonidine, lofexidine, levomethadyl acetate HCl, naltrexone, and buprenorphine; anti-cocaine agents including, but not limited to, desipramine, amantadine, fluoxidine, and buprenorphine; anti-lysergic acid diethylamide ("anti-LSD") agent including but not limited to, diazepam; anti-1-(1-phenylcyclohexyl)piperidine ("anti-PCP") agent including, but not limited to, haloperidol.

In further embodiments of combinatorial formulations and coordinate treatment methods provided herein, the secondary therapeutic agent is an anti-inflammatory agent. Examples of useful anti-inflammatory agents included, but are not limited to celecoxib, ibuprofen, ketoprofen, naproxen sodium, piroxicam, sulindac, aspirin, and nabumetone.

Suitable routes of administration for all drugs, pharmaceutical compositions and formulations comprising active ampakines (alone or combined with a secondary therapeutic agent) include, but are not limited to, oral, buccal, nasal, aerosol, topical, transdermal, mucosal, injectable, slow release, controlled release, iontophoresis, sonophoresis, and other conventional drug delivery routes, devices and methods. Injectable delivery methods are also contemplated, including but not limited to, intravenous, intramuscular, intraperitoneal, intraspinal, intrathecal, intracerebroventricular, intraarterial, and subcutaneous injection.

Examples of suitable effective unit dosage amounts of active ampakines, including anti-ADHD ampakines, for mammalian subjects may range from about 1 mg to about 1800 mg, about 2 mg to about 1400 mg, about 5 mg to about 1200 mg, about 10 mg to about 1000 mg, about 25 mg to about 800 mg, about 50 mg to about 600 mg, or about 150 mg to about 400 mg. The effective dosage will be selected within narrower ranges of, for example, about 1 mg to 10 mg, 5 mg to 10 mg, 10 mg to 25 mg, 30 mg to 50 mg, 50 mg to 100 mg, 75 mg to 150 mg, 100 mg to 200 mg, or about 250 mg to 500 mg. These and other effective unit dosage amounts may be administered in a single dose, or in the form of multiple daily, weekly or monthly doses, for example in a dosing regimen comprising from 1 to 4, or 2-3, doses administered per day, per week, or per month. Dosages of about 2 mg, 5 mg, 10 mg, 25 mg, 50 mg, 75 mg, 100 mg, 150 mg, 200 mg, 300 mg, or 500 mg, are administered one, two, three, or four times per day. Dosages of about 5-10 mg, 25-50mg, 50-75 mg, 100-200 mg, or about 250-400 mg are administered once or twice daily. Dosages are calculated based on body weight, and may be administered, for example, in amounts from about 0.01 mg/kg to about 20 mg/kg per day, 0.1 mg/kg to about 15 mg/kg per day, 0.3 mg/kg to about 10 mg/kg per day, 1 mg/kg to about 20 mg/kg per day, 2 mg/kg to about 10 mg/kg per day or 3 mg/kg to about 15 mg/kg per day.

The amount, timing, and mode of delivery of the ampakine compositions of will be routinely adjusted on an individual basis, depending on such factors as weight, age, gender, and condition of the individual, the acuteness of the condition to be treated and/or related symptoms, whether the administration is prophylactic or therapeutic, and on the basis of other factors known to effect drug delivery, absorption, pharmacokinetics, including half-life, and efficacy. An effective dose or multi-dose treatment regimen for the compounds of the invention will ordinarily be selected to approximate a minimal dosing regimen that is necessary and sufficient to substantially prevent or alleviate one or more symptom(s) of the targeted CNS condition in the subject, for example one or more symptoms of ADHD. Effective dosages of anti-CNS disorder ampakines will yield at least a 10%, 20%, 30%, 50% or greater reduction, up to a 75-90%, or 95% or greater, reduction, in one or more symptoms associated with the targeted CNS condition (for example one or more symptoms of ADHD), compared to placebo-treated or other suitable control subjects.

Pharmaceutical dosage forms of a compound of the present invention may optionally include excipients recognized in the art of pharmaceutical compounding as being suitable for the preparation of dosage units as discussed herein. Such excipients include, without intended limitation, binders, fillers, lubricants, emulsifiers, suspending agents, sweeteners, flavorings, preservatives, buffers, wetting agents, disintegrants, effervescent agents and other conventional excipients and additives.

Ampakines effective as anti-CNS disorder drugs (e.g., anti-ADHD drugs), as well as all "secondary therapeutic agents" contemplated herein, may be provided or formulated as pharmaceutically acceptable salts (e.g., inorganic and organic acid addition salts). These can include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; organic acid salts such as acetate, citrate, lactate, succinate, tartrate, maleate, fumarate, mandelate, acetate, dichloroacetate, trifluoroacetate, oxalate, formate and the like; sulfonates such as methanesulfonate, benzenesulfonate, p-toluenesulfonate and the like; and amino acid salts such as arginate, asparginate, glutamate, tartrate, gluconate and the like.

Ampakines and secondary therapeutic agents for use within treatment methods of the invention, including ADHD treatment methods, will often be formulated and administered in an oral dosage form, optionally in combination with a carrier or other additive(s). Suitable carriers common to pharmaceutical formulation technology include, but are not limited to, microcrystalline cellulose, lactose, sucrose, fructose, glucose dextrose, or other sugars, di-basic calcium phosphate, calcium sulfate, cellulose, methylcellulose, cellulose derivatives, kaolin, mannitol, lactitol, maltitol, xylitol, sorbitol, or other sugar alcohols, dry starch, dextrin, maltodextrin or other polysaccharides, inositol, or mixtures thereof. Exemplary unit oral dosage forms for use in this invention include tablets and capsules, which may be prepared by any conventional method of preparing pharmaceutical oral unit dosage forms can be utilized in preparing oral unit dosage forms. Oral unit dosage forms, such as tablets or capsules, may contain one or more conventional additional formulation ingredients, including, but are not limited to, release modifying agents, glidants, compression aides, disintegrants, lubricants, binders, flavors, flavor enhancers, sweeteners and/or preservatives. Suitable lubricants include stearic acid, magnesium stearate, talc, calcium stearate, hydrogenated vegetable oils, sodium benzoate, leucine carbowax, magnesium lauryl sulfate, colloidal silicon dioxide and glyceryl monostearate. Suitable glidants include colloidal silica, fumed silicon dioxide, silica, talc, fumed silica, gypsum and glyceryl monostearate. Substances which may be used for coating include hydroxypropyl cellulose, titanium oxide, talc, sweeteners and colorants. The aforementioned effervescent agents and disintegrants are useful in the formulation of rapidly disintegrating tablets known to those skilled in the art. These typically disintegrate in the mouth in less than one minute, and preferably in less than thirty seconds. By effervescent agent is meant a couple, typically an organic acid and a carbonate or bicarbonate.

Alternate formulations of ampakines and secondary therapeutic agents are provided for parenteral administration, including aqueous and non-aqueous sterile injection solutions which may optionally contain anti-oxidants, buffers, bacteriostats and/or solutes which render the formulation isotonic with the blood of the mammalian subject; aqueous and non-aqueous sterile suspensions which may include suspending agents and/or thickening agents; dispersions; and emulsions. The formulations may be presented in unit-dose or multi-dose containers. Pharmaceutically acceptable formulations and ingredients will typically be sterile or readily sterilizable, biologically inert, and easily administered. Parenteral preparations typically contain buffering agents and preservatives, and may be lyophilized for reconstitution at the time of administration. Parental formulations may also include polymers for extended release following parenteral administration. Such polymeric materials are well known to those of ordinary skill in the pharmaceutical compounding arts. Extemporaneous injection solutions, emulsions and suspensions may be prepared from sterile powders, granules and tablets of the kind previously described. Preferred unit dosage formulations are those containing a daily dose or unit, daily sub-dose, as described herein above, or an appropriate fraction thereof, of the active ingredient(s).

Within exemplary compositions and dosage forms of the invention, it is often desirable to achieve extended plasma drug levels, which may include the use of a sustained release formulation. In exemplary formulations, a sustained release composition provides therapeutically effective plasma levels of the ampakine (and/or secondary therapeutic agent) over a sustained delivery period of approximately 8 hours or longer, and in some instances for a sustained delivery period of approximately 12 hours, 16 hours, 18 hours or longer, up to a sustained delivery period of approximately 24 hours or longer.

In exemplary embodiments, an anti-ADHD ampakine as described herein is combined with a sustained release vehicle, matrix, binder, or coating material. As used herein, the term "sustained release vehicle, matrix, binder, or coating material" refers to any vehicle, matrix, binder, or coating material that effectively, significantly delays dissolution of the active compound *in vitro,* and/or delays, modifies, or extends delivery of the active compound into the blood stream (or other *in vivo* target site of activity) of a subject following administration (e.g., oral administration), in comparison to dissolution and/or delivery provided by an "immediate release" formulation, as described herein, of the same dosage amount of the active compound. Accordingly, the term "sustained release vehicle, matrix, binder, or coating material" as used herein is intended to include all such vehicles, matrices, binders and coating materials known in the art as "sustained release", "delayed release", "slow release", "extended release", "controlled release", "modified release", and "pulsatile release" vehicles, matrices, binders and coatings.

In one aspect of the invention, an oral sustained release dosage composition is provided for administering an active anti-CNS disorder (e.g., anti-ADHD) ampakine via a sustained, delayed, gradual or modified release delivery mode into a gastrointestinal compartment (e.g., the intestinal lumen) of the subject. A therapeutic concentration of the ampakine is thereby maintained in a blood plasma, tissue, organ or other target site of activity (e.g., a central nervous system tissue, fluid or compartment) in the patient. When following this method, the side effect profile of the ampakine (and any secondary therapeutic agent) is less severe than a side effect profile of an equivalent dose of the drug(s) administered in an immediate release oral dosage form. The drug(s) are delivered into the blood plasma or other target site of activity in the subject at a sustained therapeutic level over a period of at least about 6 hours, often over a period of at least about 8 hours, at least about 12 hours, or at least about 18 hours, and in other embodiments over a period of about 24 hours or greater. By sustained therapeutic level is meant a plasma concentration level of at least a lower end of a therapeutic dosage range as determined by clinical observations. In more detailed embodiments of the invention, the sustained release compositions and dosage forms comprise the anti-CNS disorder ampakine in a desired dosage amount (e.g., about 5 mg, 10 mg, 25 mg, 50 mg, 100 mg, 200 mg, 400 mg, 600 mg, or 800 mg) that, following administration to a mammalian subject, yields a minimum plasma concentration of at least a minimum determined therapeutic dosage range over a period of at least about 6 hours, at least about 8 hours, at least about 12 hours, at least about 18 hours, or up to 24 hours or longer. In more detailed embodiments the sustained release compositions and dosage forms yield a minimum plasma concentration that is known to be associated with a mid-high therapeutic dosage range, correlated with sustained clinical efficacy over a period of at least about 6 hours, at least about 8 hours, at least about 12 hours, at least about 18 hours, up to 24 hours or longer.

In certain embodiments, the anti-CNS disorder ampakine is administered in a dosage form that mediates delivery of the ampakine into the blood plasma or other target site of activity in the subject (including, but not limited to, areas of the brain such as the prefrontal cortex, frontal cortex, thalamus, striatum, ventral tegmental area, other cortical areas, hippocampus, hypothalamus, or nucleus accumbens) in a sustained release profile, characterized by having from 0% to 20% of the active ampakine is released and delivered (e.g., as determined by measuring blood plasma levels) within in 0 to 2 hours, from 20% to 50% of the active compound released and delivered within about 2 to 12 hours, from 50% to 85% of the active compound is released and delivered within about 3 to 20 hours, and greater than 75% of the active compound is released and delivered within about 5 to 18 hours.

In related embodiments, sustained oral or intravenous delivery of the anti-CNS disorder ampakine yields a curve of plasma concentration having an area under the curve which is approximately proportional to the dose, and a maximum concentration (Cₘₐₓ) that is approximately proportional to the dose. In other embodiments, the Cₘₐₓ is less than about 80%, often less than about 75%, in some embodiments less than about 60%, or 50%, of a Cₘₐₓ obtained after administering an equivalent dose of the active compound in an immediate release oral or intravenous dosage form. As used herein, the term "immediate release oral dosage form" refers to a dosage form wherein the active compound readily dissolves upon contact with a liquid physiological medium, for example phosphate buffered saline (PBS) or natural or artificial gastric fluid. In certain embodiments, an immediate release formulation will be characterized in that at least 70% of the active compound will be dissolved within a half hour after the dosage form is contacted with a liquid physiological medium. In alternate embodiments, at least 80%, 85%, 90% or more, or up to 100%, of the active compound in an immediate release dosage form will dissolve within a half hour following contact of the dosage form with a liquid physiological medium in an art-accepted *in vitro* dissolution assay. These general characteristics of an immediate release dosage form will often relate to powdered or granulated compositions or a capsulated dosage form, for example a gelatin-encapsulated dosage form, where dissolution will often be relatively immediate after dissolution/failure of the gelatin capsule. In alternate embodiments, the immediate release dosage form may be provided in the form of a compressed tablet, granular preparation, powder, or even liquid dosage form, in which cases the dissolution profile will often be even more immediate (e.g., wherein at least 85%-95% of the active compound is dissolved within a half hour).

Sustained release dosage forms of the present invention can take any form as long as one or more of the dissolution, release, delivery and/or pharmacokinetic property(ies) identified above are satisfied. Within illustrative embodiments, the composition or dosage form can comprise an anti-CNS disorder ampakine combined with any one or combination of: a drug-releasing polymer, matrix, bead, microcapsule, or other solid drug-releasing vehicle; drug-releasing tiny timed-release pills or mini-tablets; compressed solid drug delivery vehicle; controlled release binder; multi-layer tablet or other multi-layer or multi-component dosage form; drug-releasing lipid; drug-releasing wax; and a variety of other sustained drug release materials as contemplated herein, or formulated in an osmotic dosage form. Sustained release vehicles, matrices, binders and coatings for use in accordance with the invention include any biocompatible sustained release material which is inert to the active agent and which is capable of being physically combined, admixed, or incorporated with the active compound. Useful sustained release materials may be dissolved, degraded, disintegrated, and/or metabolized slowly under physiological conditions following delivery (e.g., into a gastrointestinal tract of a subject, or following contact with gastric fluids or other bodily fluids). Useful sustained release materials are typically non-toxic and inert when contacted with fluids and tissues of mammalian subjects, and do not trigger significant adverse side effects such as irritation, immune response, inflammation, or the like. They are typically metabolized into metabolic products which are biocompatible and easily eliminated from the body. Exemplary sustained release compositions may include, for example, ethylcellulose, hydroxyethyl cellulose; hydroxyethylmethyl cellulose; hydroxypropyl cellulose; hydroxypropylmethyl cellulose; hydroxypropylmethyl cellulose phthalate; hydroxypropylmethylcellulose acetate succinate; hydroxypropylmethylcellulose acetate phthalate; sodium carboxymethylcellulose; cellulose acetate phthalate; cellulose acetate trimellitate; polyoxyethylene stearates; polyvinyl pyrrolidone; polyvinyl alcohol; copolymers of polyvinyl pyrrolidone and polyvinyl alcohol; polymethacrylate copolymers; and mixtures thereof. Additional polymeric materials for use as sustained release vehicles, matrices, binders, or coatings (some operable in intravenous or intraperitoneal formulations) may include cellulose ethers, co polymeric and homopolymeric polyesters with hydrolysable ester linkages, polyglycolic acids (PGAs), polylactic acids (PLAs), poly(DL-lactic acid-co-glycolic acid)(DL PLGA), poly(D-lactic acid-co glycolic acid)(D PLGA) and poly(L-lactic acid-co-glycolic acid)(L PLGA), poly(ε-caprolactone), poly(ε-aprolactone-CO-lactic acid), poly(ε-aprolactone-CO-glycolic acid), poly(B-hydroxy butyric acid), poly(alkyl-2-cyanoacrilate), hydrogels such as poly(hydroxyethyl methacrylate), polyamides, poly-amino acids (e.g., poly-L-leucine, poly-glutamic acid, poly-L-aspartic acid, and the like), poly (ester ureas), poly (2-hydroxyethyl DL-aspartamide), polyacetal polymers, polyorthoesters, polycarbonates, polymaleamides, polysaccharides, and copolymers thereof. Methods for preparing pharmaceutical formulations using these and other materials are generally known to those skilled in the art.

Pharmaceutical compositions and dosage forms of the invention may include erodible or no erodible polymers in multi-layer dosage forms. Active ampakine compounds can be coated onto a polymer such as a polypeptide, collagen, gelatin, polyvinyl alcohol, polyorthoester, polyacetyl, or a polyorthocarbonate, and the coated polymer folded onto itself to provide a bilaminated dosage form. In operation, the bioerodible dosage form erodes at a controlled rate to dispense the active compound over a sustained release period. Representative biodegradable polymers for use in this and other aspects of the invention can be selected from, for example, biodegradable poly(amides), poly (amino acids), poly(esters), poly(lactic acid), poly(glycolic acid), poly(carbohydrate), poly(orthoester), poly (orthocarbonate), poly(acetyl), poly(anhydrides), biodegradable poly(dehydropyrans), and poly(dioxinones).

Ampakine drug delivery can further be controlled by loading the active drug into a polymer that releases the drug by diffusion through the polymer, or by flux through pores or by rupture of a polymer matrix. These dosage forms can be manufactured by procedures known in the art, for example by blending a pharmaceutically acceptable carrier like polyethylene glycol, with a pre-determined dose of the active compound(s) at an elevated temperature (e.g., 37°C), and adding it to a silastic medical grade elastomer with a cross-linking agent, for example, octanoate, followed by casting in a mold. The step is repeated for each optional successive layer. The system is allowed to set for 1 hour, to provide the dosage form. Representative polymers for manufacturing such sustained release dosage forms include, but are not limited to, olefin, and vinyl polymers, addition polymers, condensation polymers, carbohydrate polymers, and silicon polymers as represented by polyethylene, polypropylene, polyvinyl acetate, polymethylacrylate, polyisobutylmethacrylate, poly alginate, polyamide and polysilicon. These polymers and procedures for manufacturing them are well known in the art.

In additional embodiments of the invention sustained release of the active ampakine is mediated by incorporation of the drug within microbeads, tiny pills or mini-tablets. The beads, tiny pills or mini-tablets may comprise a hydrophilic polymer selected from the group consisting of a polysaccharide, agar, agarose, natural gum, alkali alginate including sodium alginate, carrageenan, fucoidan, furcellaran, laminaran, hypnea, gum arabic, gum ghatti, gum karaya, gum tragacanth, locust bean gum, pectin, amylopectin, gelatin, and a hydrophilic colloid. The hydrophilic polymer may be formed into a plurality (e.g., 4 to 50) tiny pills or mini-tablets, wherein each tiny pill or mini-tablet comprises a pre-determined partial dose e.g., a dose of about 10 ng, 0.5 mg, 1 mg, 1.2 mg, 1.4 mg, 1.6 mg, 5.0 mg etc. The tiny pills and mini-tablets may further comprise a release rate-controlling wall of 0.001 up to 10 mm thickness to provide for timed release of the active compound. Representative wall forming materials include a triglyceryl ester selected from the group consisting of glyceryl tristearate, glyceryl monostearate, glyceryl dipalmitate, glyceryl laureate, glyceryl didecenoate and glyceryl tridenoate. Other wall forming materials comprise polyvinyl acetate, phthalate, methylcellulose phthalate and microporous olefins. The beads, tiny pills and mini-tablets may further comprise a blend of particles of different sizes and/or release properties, and the particles may be contained in a hard gelatin or non-gelatin capsule or soft gelatin capsule. Procedures for manufacturing tiny pills and mini-tablets are well known in the art.

In other embodiments of the invention, drug-releasing lipid matrices are used to formulate ampakine compositions and dosage forms. In one example, solid microparticles of the active compound are coated with a thin controlled release layer of a lipid (e.g., glyceryl behenate and/or glyceryl palmitostearate), and the lipid-coated particles can optionally be compressed to form a tablet. In related embodiments, drug-releasing waxes can be used for producing sustained release compositions and dosage forms, including for example carnauba wax, candedilla wax, esparto wax, ouricury wax, hydrogenated vegetable oil, bees wax, paraffin, ozokerite, castor wax, and mixtures thereof.

Osmotic delivery systems are also provided for sustained release delivery of anti-CNS disorder (e.g., anti-ADHD) ampakines. Exemplary osmotic delivery systems deliver the active compound by imbibing fluid through a semipermeable wall at a fluid imbibing rate determined by the permeability of the semipermeable wall and the osmotic pressure across the semipermeable wall, causing a push layer to expand, thereby delivering the active compound through an exit passageway to a patient over a prolonged period of time (up to 24 or even 30 hours). These dosage forms and their construction are well known in the art.

In other embodiments an anti-CNS disorder ampakine is encapsulated for controlled delivery in microcapsules, microparticles, or microspheres, prepared, for example, by coacervation techniques or by interfacial polymerization, for example, hydroxymethylcellulose or gelatin-microcapsules and poly(methylmethacylate) microcapsules, respectively, in colloidal drug delivery systems (for example, liposomes, albumin microspheres, microemulsions, nano-particles and nanocapsules) or in macroemulsions. In exemplary embodiments, microparticles are formed, for example, by encapsulating the active ampakine within a biocompatible, biodegradable wall-forming material (e.g., a polymer), to provide sustained or delayed release of the active compound. In these methods, the active compound is typically dissolved, dispersed, or emulsified in a solvent containing the wall forming material. Solvent is then removed from the microparticles to form the finished micro particle product. Microencapsulation processes are well known and can be routinely implemented to prepare micro particles containing active ampakines described herein.

In yet additional embodiments, enteric-coated preparations can be used for oral sustained release administration of anti-CNS disorder ampakines. Preferred coating materials include polymers with a pH-dependent solubility (i.e., pH-controlled release), polymers with a slow or pH-dependent rate of swelling, dissolution or erosion (i.e., time-controlled release), polymers that are degraded by enzymes (i.e., enzyme-controlled release) and polymers that form firm layers that are destroyed by an increase in pressure (i.e., pressure-controlled release). Enteric coatings may function as a means for mediating sustained release of ampakines by providing one or more barrier layers, which may be located entirely surrounding the active compound, between layers of a multi-layer solid dosage form, and/or on one or more outer surfaces of one or multiple layers of a multi-layer solid dosage form (e.g., on end faces of layers of a substantially cylindrical tablet). Such barrier layers may, for example, be composed of polymers which are either substantially or completely impermeable to water or aqueous media, or are slowly erodible in water or aqueous media or biological liquids and/or which swell in contact with water or aqueous media. Suitable polymers for use as a barrier layer include acrylates, methacrylates, copolymers of acrylic acid, celluloses and derivatives thereof such as ethyl celluloses, cellulose acetate propionate, polyethylenes and polyvinyl alcohols etc. Barrier layers comprising polymers which swell in contact with water or aqueous media may swell to such an extent that the swollen layer forms a relatively large swollen mass, the size of which delays its immediate discharge from the stomach into the intestine. The barrier layer may itself contain active material content, for example the barrier layer may be a slow or delayed release layer. Barrier layers may typically have an individual thickness of 10 microns up to 2 mm. Suitable polymers for barrier layers which are relatively impermeable to water include the Methocel^{™} series of polymers, used singly or combined, and Ethocel^{™} polymers.

Additional coating materials for use in constructing solid dosage forms to mediate sustained release of ampakines include, but are not limited to, polyethylene glycol, polypropylene glycol, copolymers of polyethylene glycol and polypropylene glycol, poly(vinylpyrrolidone), ethyl cellulose, hydroxyethyl cellulose, hydroxypropyl cellulose, carboxymethyl cellulose, carboxymethylethyl cellulose, starch, dextran, dextrin, chitosan, collagen, gelatin, bromelain, cellulose acetate, unplasticized cellulose acetate, plasticized cellulose acetate, reinforced cellulose acetate, cellulose acetate phthalate, cellulose acetate trimellitate, hydroxypropylmethylcellulose, hydroxypropylmethyl-cellulose phthalate, hydroxypropylmethylcellulose acetate succinate, hydroxypropylmethylcellulose acetate trimellitate, cellulose nitrate, cellulose diacetate, cellulose triacetate, agar acetate, amylose triacetate, beta glucan acetate, beta glucan triacetate, acetaldehyde dimethyl acetate, cellulose acetate ethyl carbamate, cellulose acetate phthalate, cellulose acetate methyl carbamate, cellulose acetate succinate, cellulose acetate dimethaminoacetate, cellulose acetate ethyl carbonate, cellulose acetate chloroacetate, cellulose acetate ethyl oxalate, cellulose acetate methyl sulfonate, cellulose acetate butyl sulfonate, cellulose acetate propionate, cellulose acetate p-toluene sulfonate, triacetate of locust gum bean, cellulose acetate with acetylated hydroxyethyl cellulose, hydroxlated ethylene-vinyl acetate, cellulose acetate butyrate, polyalkenes, polyethers, polysulfones, polyethersulfones, polystyrenes, polyvinyl halides, polyvinyl esters and ethers, natural waxes and synthetic waxes.

In further detailed embodiments of the invention, sustained release ampakine dosage forms are provided by formulating the active ampakine compound in multi-layer tablet or other multi-layer or multi-component dosage form. In exemplary embodiments, the active compound is formulated in layered tablets, for example having a first layer which is an immediate release layer and a second layer which is a slow release layer. Other multi-layered dosage forms of the invention may comprise a plurality of layers of compressed active ingredient having variable (i.e., selectable) release properties selected from immediate, extended and/or delayed release mechanisms. Multi-layered tablet technologies useful to produce sustained, or bimodal, release dosage forms are well known in the art. Other multi-component dosage forms for providing sustained or bimodal delivery include tablet formulations having a core containing the active compound coated with a release retarding agent and surrounded by an outer casing layer. The release retarding agent is typically an enteric coating as described above, yielding immediate release of contents of the outer core, followed by a second phase from the core which is delayed until the core reaches the intestine. Immediate release layers may be, for example, layers which disintegrate immediately or rapidly, or swellable polymers that swell immediately and extensively in contact with water or aqueous media to immediately release active material. Slow release layers may comprise release retarding vehicles, matrices, binders, coatings, or excipients, for example pH sensitive polymers (e.g., methacrylic acid copolymers) and other release-retarding polymers, including gellable polymers (e.g., methylcellulose, carboxymethylcellulose, low-molecular weight hydroxypropylmethylcellulose, low-molecular weight polyvinyl alcohols, polyoxyethyleneglycols, non-cross linked polyvinylpyrrolidone) and cross-linked polymers (e.g., cross-linked sodium carboxymethylcellulose, cross-linked hydroxypropylcellulose, high-molecular weight hydroxypropylmethylcellulose, carboxymethylamide, potassium methacrylatedivinylbenzene co-polymer, polymethylmethacrylate, cross-linked polyvinylpyrrolidone, high-molecular weight polyvinyl alcohols, etc.

The pharmaceutical compositions and dosage forms of the current invention will typically be provided for administration in a sterile or readily sterilizable, biologically inert, and easily administered form.

In other embodiments the invention provides pharmaceutical kits for treating a CNS cognitive disorder, psychiatric disorder, behavioral disorder or attention deficit disorder, for example ADHD. The kits comprise an anti-CNS disorder ampakine, wherein the ampakine is 1-(benzofurazan-5-ylcarbonyl)morpholine (CX717) or N-Methyl-N-tetrahydro-2H-pyran-4-yl-[2,1,3]-benzoxadiazole-5-carboxarnide (CX1739), and anti-ADHD ampakine and a container means for containing the ampakine in a sterile and stable form for administration to the subject (for example a conventional pharmaceutical container, divided bottle, or foil pack). The container means can include a package bearing a label or insert that provides instructions for single or multiple use of the kit to treat the target CNS disorder and reduce symptoms of the targeted CNS disorder in the subject. In exemplary embodiments, an anti-ADHD ampakine is formulated in a pharmaceutical composition, in a single or multiple dosage form (for example a liquid or solid oral dosage form) in the kit, which includes appropriate labeling and use instructions (e.g., specifying dosing, side effects, and use for treating ADHD).

The skilled artisan will understand this invention is not limited to the particular compounds, formulations, process steps, and materials disclosed herein, as such compounds, formulations, process steps, and materials are provided for illustrative purposes only. The description provided here, following the discoveries and teachings of the invention as a whole, can be changed and expanded in equivalent form and purpose by the skilled artisan, without undue experimentation. Likewise, the terminology employed herein is exemplary only, to describe illustrative embodiments. The following examples are provided for the same, illustrative and non-limiting purpose.

### Example I.

### Identification and Characterization of Low Impact Ampakines

### For Selection and Use as Anti-ADHD Drugs

Within the following Examples two exemplary ampakines were identified and characterized for their novel utility and performance in accepted in vitro and animal models of ADHD drug efficacy in humans. These exemplary compounds, "CX717" and "CX1739", are confirmed useful for anti-ADHD clinical use in human subjects. Additional compounds are likewise identified and selected as useful anti-ADHD drug candidates, as described.

CX717 has the chemical name 1-(benzofurazan-5-ylcarbonyl)morpholine, and corresponds to the following structure.

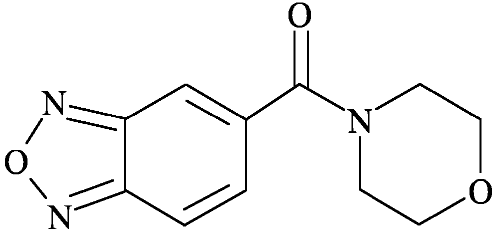

CX1739 has the chemical name N-Methyl-N-tetrahydro-2H-pyran-4-yl-[2,1,3]-benzoxadiazole-5-carboxamide, and corresponds to the following structure.

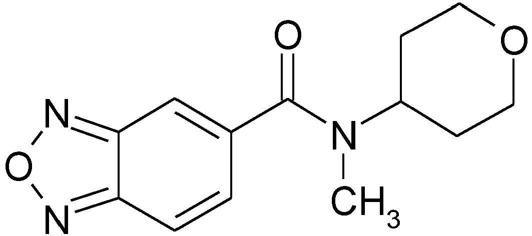

These two compounds correspond to an exemplary class of ampakines from which these and other exemplary anti-ADHD drug candidates have been selected and demonstrated to be therapeteutically (anti-ADHD) effective according to the teachings of the invention. Following these teachings, many additional active drug candidates and therapeutically effective anti-ADHD compounds can be selected from other classes of ampakines as described herein, without undue experimentation.

CX717 and CX 1739 were selected as illustrative anti-ADHD drug candidates in part for their desirable clinical characteristics as "low impact" ampakines. As used herein, "low impact ampakine" refers to ampakines having substantial potency for treating ADHD and related conditions (e.g., as determined using art-accepted in vitro and animal models for ADHD), with a substantially improved PK/PD profile compared to previously-studied ampakines (e.g., CX516, and CX614), and having little or no convulsant activities at therapeutic dosage levels.

Convulsant activity and several other drug activity characteristics were screened and determined qualitatively and qualitatively for CX717 and CX1739 in a diverse assemblage of art-accepted models. An illustrative panel of assays employed within the invention is generally depicted in Tables 1 and 2, below. These representative studies can be used to identify additional compounds with low convulsant activity and novel efficacy, as described here for the exemplary compounds, CX717 and CX1739.

**Table 1. Pharmacodynamic Study Summary for CX717**

| **Type of Study** | **Species (strain)** | **Route** | **Dose Range (mg/kg)** | **Minimum Effective Dose (mg/kg)** |
|---|---|---|---|---|
| Effect on evoked fEPSPs in rat hippocampus | Rat (Long Evans) | IP | 5-20 | 5 |
| Effect on induction of LTP in rat hippocampus | Rat (Long Evans) | IP | 2 | 2 |
| Novel object recognition memory | Rat (Wistar) | IP | 0.1 - 1.0 | 0.3 |
| Radial arm maze - spatial memory | Rat (SD) | IP | 0.03 - 10 | 0.3 |
| Delayed match-to-sample test | Monkey (Rhesus) | PO | 0.8 | 0.8 |
| Amphetamine-stimulated locomotion | Mouse (CD1) | IP | 5.6 - 30 | 18 |
| Amphetamine-stimulated locomotion and rearing | Rat (SD) | PO | 1-10 | 1 |

**Table 2. Pharmacodynamic Study Summary for CX1739**

| **Type of Study** | **Species (strain)** | **Route** | **Dose Range (mg/kg)** | **Minimum Effective Dose (mg/kg)** |
|---|---|---|---|---|
| Effect on evoked fEPSPs in rat hippocampus | Rat (Long Evans) | IP | 5-20 | 5 |
| Effect on induction of LTP in rat hippocampus | Rat (Long Evans) | IP | 1-3 | 3 |
| Novel object recognition memory | Rat (Wistar) | IP | 0.03 - 0.3 | 0.03 |
| Radial arm maze - spatial memory | Rat (SD) | IP | 0.03 - 1.0 | 0.3 |
| 5 Choice serial reaction time task | Rat (Lister) | IP | 3-10 | 3 |
| Delayed match-to-sample test | Monkey (Rhesus) | PO | 0.03 - 0.3 | 0.3 |
| Amphetamine-stimulated locomotion | Mouse (CD1) | IP | 5.6 - 30 | 30 |
| Amphetamine-stimulated locomotion and rearing | Rat (SD) | PO | 1 -10 | 1 |

### Example II.

### In Vitro Receptor and Transporter Binding (Agonist and Antagonist) Assays

For initial characterization of CX717 and CX1739, these two ampakines were evaluated in broad in vitro pharmacology screens, comprised of radioligand binding assays for 62 receptors, transporters or ion channel-associated macromolecular complexes, as shown in Table 3.

**Table 3 In Vitro Receptor Binding Assays. CX717 and CX1739 were tested for inhibition of specific binding to the following broad panel of receptors and other targets.**

| | |
|---|---|
| acetylcholine esterase | Melatonin (non-selective) |
| Adenosine, non-selective | Monoamine oxidase, MAO-A and MAO-B |
| Adrenergic, α1, α2, B (nonselective) | Muscarinic (M1, M2, non-selective central, non-selective peripheral |
| Angiotensin, AT1 and AT2 | Neurokinin, NK-1, NK-2, NK-3 |
| Bradykinin BK2 | Nicotinic (α-bungarotoxin insensitive) |
| Ca²⁺ channel, type N and L | Nitric oxide synthase (NOS) |
| Cholecystokinin, CCK1 and CCK2 | Norepinephrine transporter |
| Corticotropin releasing hormone, non-selective | Opioid (non-selective) |
| Dopamine transporter | Orphanin |
| Dopamine (non-selective) | Oxytocin |
| Endothelin ET-A, ET-B | Platelet activating factor |
| Estrogen | Potassium channel (ATP-sensitive, Ca²⁺ activated, I(Kr) hERG) |
| GABA-A, Agonist, BDZ sites | Serotonin transporter |
| GABA-B | Serotonin (non-selective) |
| Galanin, non-selective | Sigma (non-selective) |
| Glutamic acid decarboxylase | Sodium channel, site 2 |
| Glutamate (AMPA, kainate, NMDA agonist, Glycine sites) | Testosterone |
| Glycine (strychnine-sensitive) | Thromboxane A2 |
| Histamine (H1, H2, H3) | Thyrotropin releasing hormone |
| Leukotriene LTD4 | Vasoactive Intestinal Peptide, non-selective Vasopressin 1 |

Both ampakines were separately tested in standard assays for the targets listed in Table 3 at a concentration of 100 µM. CX717 did not inhibit binding to any target by greater than 50%, except acetylcholine esterase, where a functional inhibition of 53% was seen. Follow-up studies generated a Ki of 165 µM for the inhibition of acetylcholine esterase function by CX717. These findings suggest that the interaction with acetylcholine esterase is not likely to be meaningful at concentrations achieved clinically. CX1739 did not inhibit binding of a reference ligand to any receptor target by greater than 50%, except a non-selective alpha1 adrenergic receptor (67%) and a cytosolic testosterone receptor (64%). These interactions are not expected to be clinically meaningful.

Additional studies involving G-protein coupled receptor assays were conducted for CX1739 to further illustrate the scope and flexibility of binding activity profiling available for ampakine screening according to the invention. CX1739 was evaluated in a second broad in vitro pharmacology screen, comprising cell-based functional G-protein coupled receptor assays for 64 GPCRs, identified in Table 4.2.2-1 below.

**Table 4.2.2-1 Functional In Vitro GPCR Assays in which CX1739 was Tested for Agonist or Antagonist Activity at a Concentration of 30 or 37.5 µM**

| | |
|---|---|
| Adenosine, A1, A2b, A3 | Lysophospholipid, LPA1, LPA2 |
| Adrenergic, a1a, a1b, a2a, a2b, β1, β2, β3 | Muscarinic, M1, M2, M3, M4, M5 |
| Bradykinin, BK2 | Neurokinin, NK-1, NK-2, NK-3 |
| Cannabinoid, CB1, CB2 | Neuromedin U, NMU1 |
| Chemokine, CCR1, CCR2B, CCR5 | Neurotensin, NTR1 |
| Cholecystokinin, CCK1 and CCK2 | Nicotinic Acid GPR109A |
| Complement, C3a, C5a | Opioid, OPRM1 |
| Corticotropin releasing factor receptor 1, 2 | Purinergic, P2Y1 |
| Dopamine, D1, D2, D5 | Platelet activating factor |
| GnRH-R | Serotonin, 5HT1A, 5HT2A, 5HT2B, SHT2C |
| GABA-B1b | Sphingosine 1-phosphate, S1P1, S1P2, S1P3, S1P4, S1P5 |
| Galanin, GAL1, GAL2 | Vasopressin, V1A, V1B, V2 |
| Histamine, H1, H2, H3 | Neuropeptide Y, NPY2, NPY4 |
| Leukotriene, LTB4, LTD4 | |

When CX1739 was tested for GPCR agonist and antagonist activities in these 64 functional GPCR assays (at concentrations of 30 µM and 37.5 µM, respectively), this exemplary ampakine did not exhibit any significant agonist or antagonist activity directed to any of the subject targets.

### Example III.

### Electrophysiology Assays

Further selection and characterization of ampakines to identify anti-ADHD drugs useful within the invention involved electrophysiological studies (to measure "electrically evoked field excitatory postsynaptic potentials" (fEPSPs) for test compounds). CX717 and CX1739 were tested for their ability to facilitate synaptic responses in vivo in anesthetized animals following peripheral administration. Stimulating and recording electrodes were inserted into the perforant path and dentate gyrus of the hippocampus, respectively. Electrically evoked field excitatory postsynaptic responses (fEPSPs) were measured before administration of ampakines, and for approximately 2 hours following intraperitoneal administration of ampakines.

Long term potentiation Electrophysiological effects of CX717 and CX1739 were examined on hippocampal LTP measured in an anesthetized rat in vivo. Evoked potentials were measured in the hilus of the dentate gyrus following stimulation of the perforant path. The current used to elicit the evoked potential was adjusted to produce a response size of 40% of the maximal spike-free amplitude. Evoked hilar field potentials were recorded every 20 sec in response to single pulse stimulation delivered at 3 Hz to the perforant path. Once a stable baseline was established, a suboptimal tetanic protocol (4 trains at 400 Hz of 20 msec duration were delivered at 0.1 Hz) was used to produce a detrimental form of LTP (or STP). Field potential recording was then continued at the same rate and intensity of stimulation as during the baseline period, to observe the amount and duration of STP induced.

Once STP declined back to baseline (usually within 30-60 min), CX717 or CX1739 was injected i.p. and field potentials were continuously recorded every 20 sec for about 15 min. This was followed by administration of a second episode of suboptimal tetanic stimulation, identical in all aspects to the first. Again, field potential recording was continued at the same rate and intensity of stimulation as during the baseline period, to observe the amount and duration of LTP induced in the presence of either ampakine.

Slice Seizure Assay In these studies extracellular field potentials recording (Population Spikes) in hippocampal slices were used to determine convulsant activity (potential seizure liability). Transverse 300-400 µm hippocampal slices were taken from adult male Sprague-Dawley rats (4-6 week old) and incubated in a submerge slice recording chamber system for 30 to 60 min. The chamber was perfused with oxygenated artificial cerebral spinal fluid (ACSF) comprising (in mM): NaCl 124, KCl 3.0, KH2PO4 1.25, CaCl2.34, MgSO4 2.5, NaHCO3 26 and glucose 10 at room temperature. Population Spike (PS) recordings were made with glass electrodes filled with 2 M NaCl (1-5megaohm) places in the region of pyramidal cell body of CA1. Electrical stimuli were produced with twisted bipolar nichrome wire electrodes in the Schaffer-commissural fiber afferents. Population spike was induced by activating Schaffer-commissural fiber using single pulse stimulation with delivered at 0.05 Hz. The maximal amplitude of PS was determined by increase stimulating intensity till second spike appears. Then the intensity was decreased to induce a 50% to 60% of maximal response. The amplitude of PS was measured for each response and plotted against time. At same time the amplitude of second PS and/or number of PS was measured and plotted against time. The size of the second PS and numbers of PS were used to estimate the seizure potential or seizure state. With 20 to 50 min stable baseline established testing compound was perfused for 10 to 20 min at each concentration to observe the effect. The concentration of testing compound was increased step by step until second or more PS was observed. The perfusion then was switched to control ACSF and continued to record until drug effect was washed back to the baseline. CX717 and CX1739 exemplary test ampakine compounds were prepared by dissolving in DMSO and then added to ACSF.

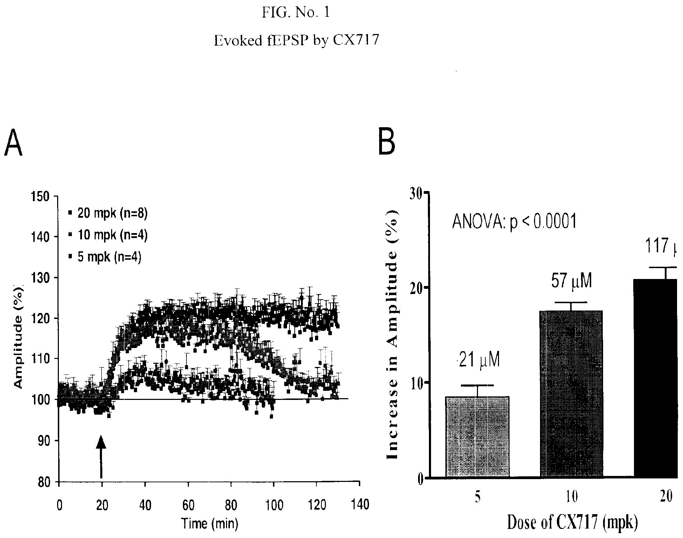

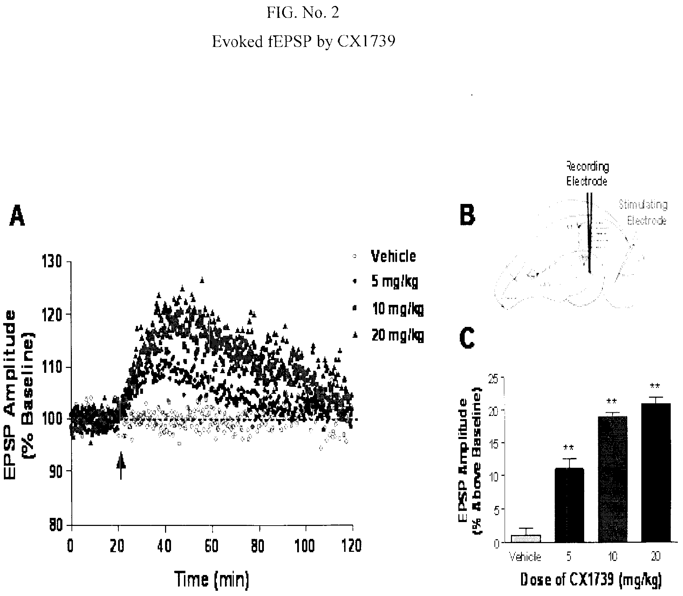

Electrically evoked field excitatory postsynaptic potentials (fEPSPs) were determined for the exemplary test ampakines CX717 and CX1739 as described. CX717 produced a long-lasting increase in the evoked fEPSP in a concentration-dependent manner (Figure 1). The response magnitude was related to the plasma levels achieved during the study. CX1739 also produced a substantial increase in EPSP amplitude (Figure 2), but the effect declined to baseline after two hours, whereas the dose of 20mg/kg CX717 plateaued for the duration of monitoring. Both ampakines appear to produce a 25% maximum increase in fEPSP amplitude.

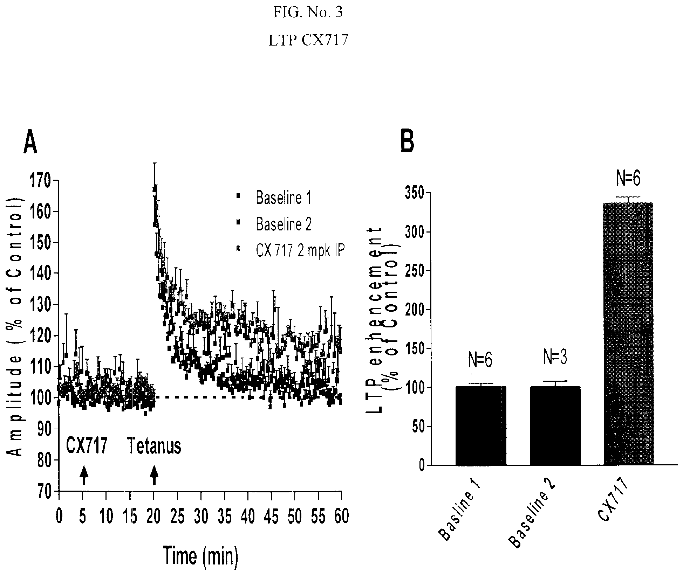

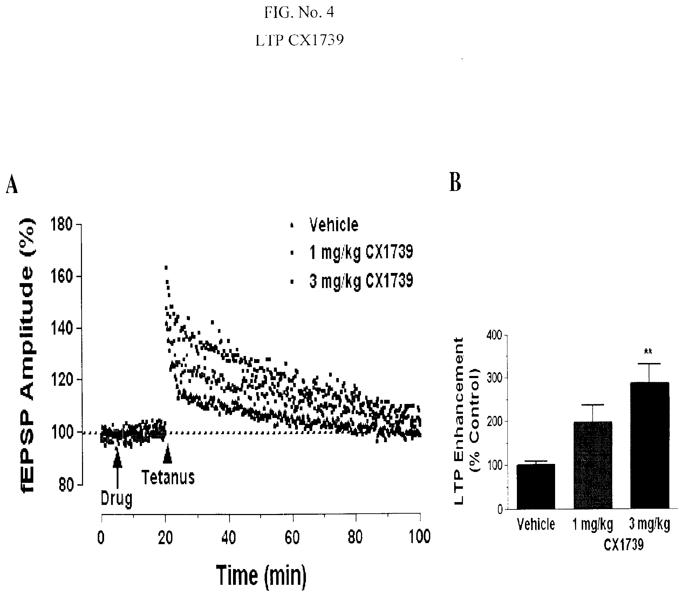

The phenomenon of long-term potentiation is considered to be the cellular model and substrate of learning and memory. It is defined as the enduring increase in the amplitude of EPSPs following high-frequency (tetanic) stimulation of afferent pathways. The effects of both exemplary test ampakines CX717 and CX1739 were examined on hippocampal LTP. Both ampakines were administered to anesthetized rats 15 minutes prior to tetanic stimulation. The study rats, and all animals employed within the studies presented herein, were housed, cared for and handled according to best standards for humane care (typically following NIH-proscribed laboratory animal care and management criteria). Neither ampakine altered baseline amplitude prior to tetanic stimulation. After the tetanus, 2mg/kg CX717 enhanced hippocampal LTP by 200% relative to baseline (P<0.01, compared to baseline 1) (Figure 3). Amplitudes of CX717-treateed rats did not return to baseline during the 40-minute monitoring period. CX1739 was tested at 1 and 3mg/kg for its effect on the amplitude of evoked potentials after tetanic stimulation. CX1739 increased fEPSP amplitudes in a dose-dependent fashion and 3mg/kg produced a statistically significant increase in LTP (p<0.01) (Figure 4).

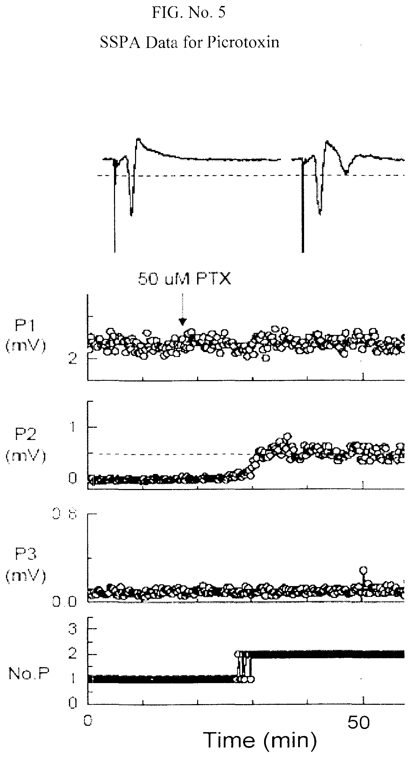

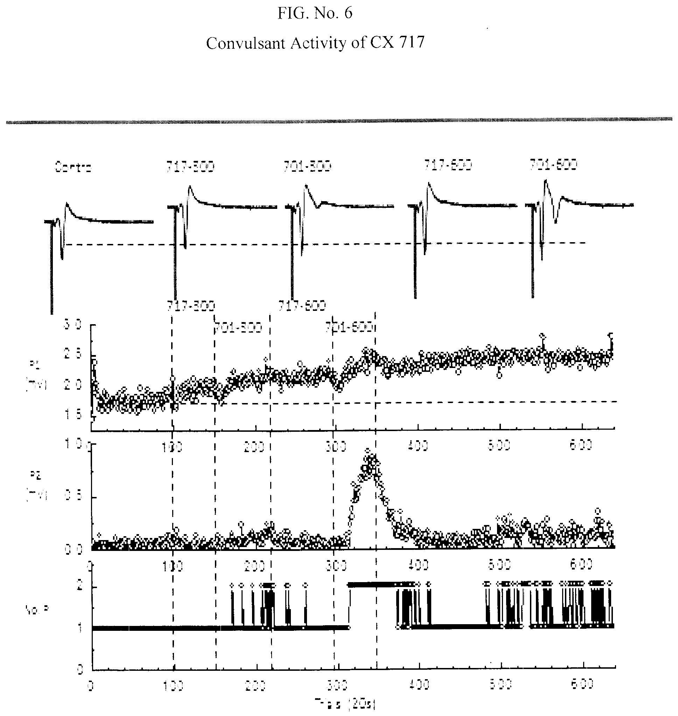

Slice Seizure Assays were conducted after 18 min stable baseline had been established, whereupon picrotoxin was perfused at 50 µM at the same speed as control solution, and after about 10 min PS (seizure activity) was be observed. Figure 5 shows the traces taken from the periods of control and 15 min after picrotoxin was washed in. The upper 3 panels of Figure 5 show amplitudes for PS 1, 2 and 3m respectively. Lower panel is the measurement of the number of PS. 50 µM picrotoxin perfusion induced a seizure like activity at the level of second PS but not third PS without increasing the amplitude of the first PS. After 60 min stable baseline had been established, CX1739 was perfused at 100 µM for 20 min and then 200 µM (limit of solubility) for another 20 min at same speed as control solution. There was no sign of seizure activity during CX1739 perfusion. (Also CX1739 did not change the amplitude of the first PS. Similarly, concentrations of CX717 up to 600uM did not display seizure activity throughout the duration of monitoring (Figure 6).

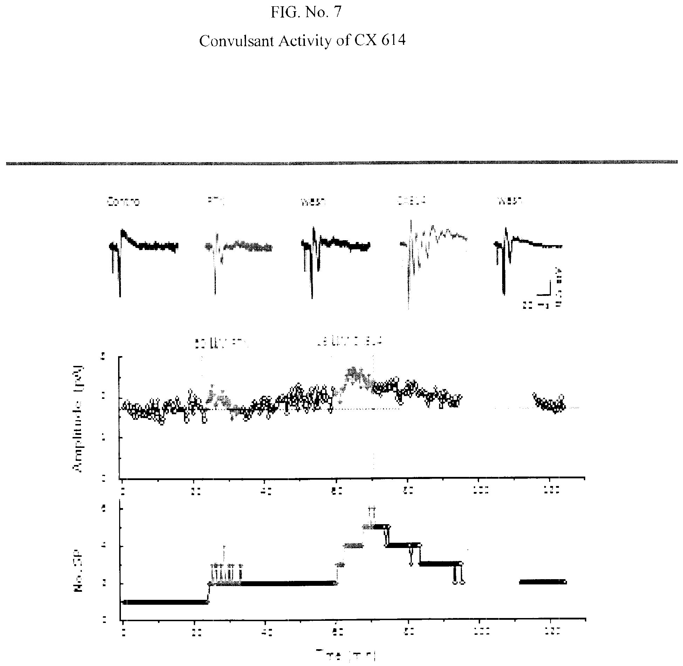

Conversely, the high-impact ampakine CX614 demonstrated seizure potential activity at 16 uM in picortoxin-induced seizure activity assays using hippocampal slice methods (Figure 7). These findings highlight major differences between these fundamentally distinct types of ampakines for use in therapeutic methods described herein.

### Example IV.

### Pharmacological Activity of Low Impact Ampakines in Diverse Animal Models

Behavioral tests of locomotor activity were conducted to determine the effects of exemplary low impact ampakines CX717 and CX1739 on normal, and amphetamine-stimulated activity in animal behavioral models. In comparable studies, the effects of CX717 and CX1739 on spontaneous locomotor activity (LMA) and rearing were determined in conventional rat models. Male Sprague-Dawley rats (175-200g; Charles River Laboratories) were given ad libitum food and water and maintained on a 12:12 hr light: dark cycle with lights on at 6:00 a.m. Each of ten test cages (standard polycarbonate animal cage; 26 cm x 48 cm x 20 cm; W x L x H) were surrounded by two photo beam arrays, placed to detect locomotor activity with a lower array and rearing behavior with an upper array. Locomotor and rearing activity were continuously monitored and recorded by computer for all ten test cages (Photo beam Activity System, San Diego Instruments, San Diego, CA). The test period was chosen as the 1st few hours of the dark period of the light: dark cycle. Approximately 15 min before the start of the dark cycle, the rats were removed from the housing facility, transported to the testing room and injected with CX717, CX1739 or vehicle and placed into photo beam activity cages. Immediately after all rats were injected the room lights were turned off and spontaneous activity data were collected for the next four hours. CX717 and CX1739 groups (0.1 to 10 mg/kg) were compared to the vehicle group.

Effects of exemplary low impact ampakines were also evaluated in mice for the ability to inhibit amphetamine-stimulated locomotor activity. Mice were placed in the locomotor chamber for 20 min to habituate, removed, injected with either vehicle or anti-ADHD ampakine (CX717), and 5 min later injected with 2 mg/kg i.p. Amphetamine. Mice were returned to the locomotor chamber 10 min later, and their activity measured for a further 15 min. In related studies, adult male CD1 mice or male Sprague-Dawley rats were acclimated in a test chamber for 20 minutes removed and dosed i.p. (mice) or orally (rats) with CX1739, vehicle, or a reference compound. Five (mice) or 10 (rats) were then injected with amphetamine (2 mg/kg, i.p.) or saline vehicle and were returned to the activity test chamber.

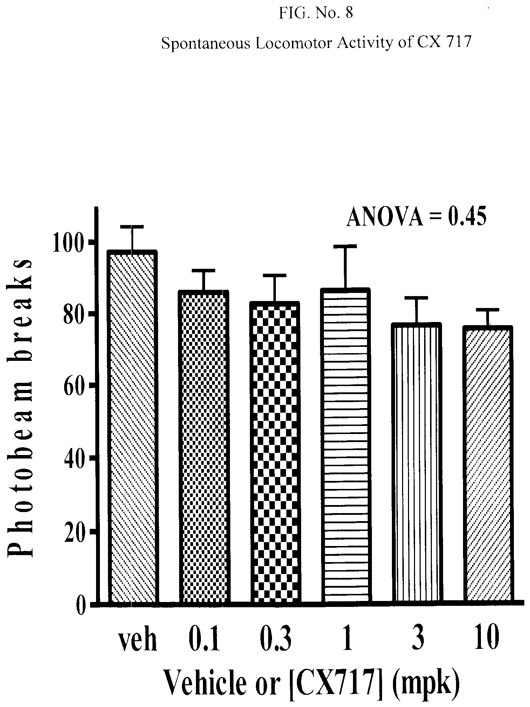

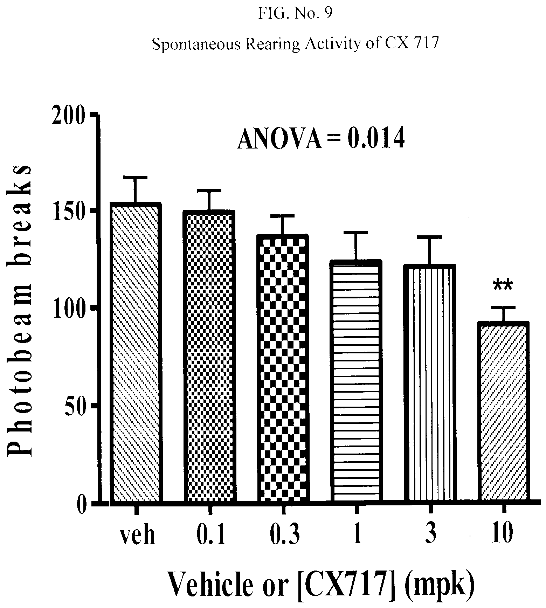

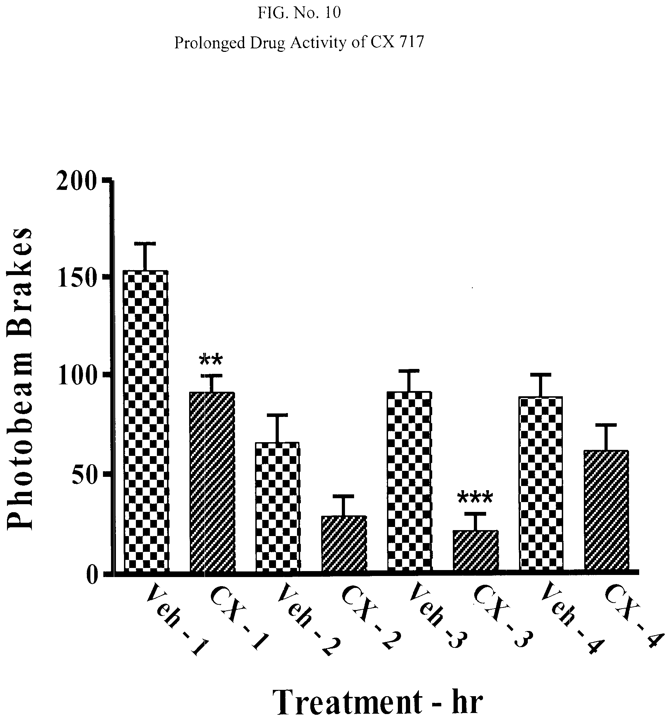

CX717 had no effect on locomotor activity during the first hour following ip administration (ANOVA = 0.45) (Figure 8). In contrast, rearing behavior decreased in a dose-dependent manner (with 10mg/kg CX717) producing a significant decrease in spontaneous rearing (ANOVA = 0.014; 10 mpk dose vs. vehicle, p < 0.01 by post-hoc Dunnett's multiple comparison test) (Figure 9). Furthermore, the pharmacodynamics observed for the effect of CX717 (10 mpk) on rearing behavior indicates a clinically desired, prolonged action of the drug (Figure 10).

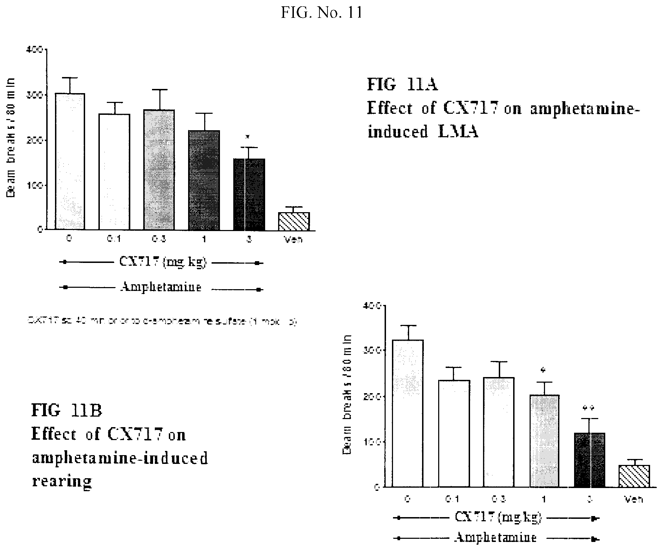

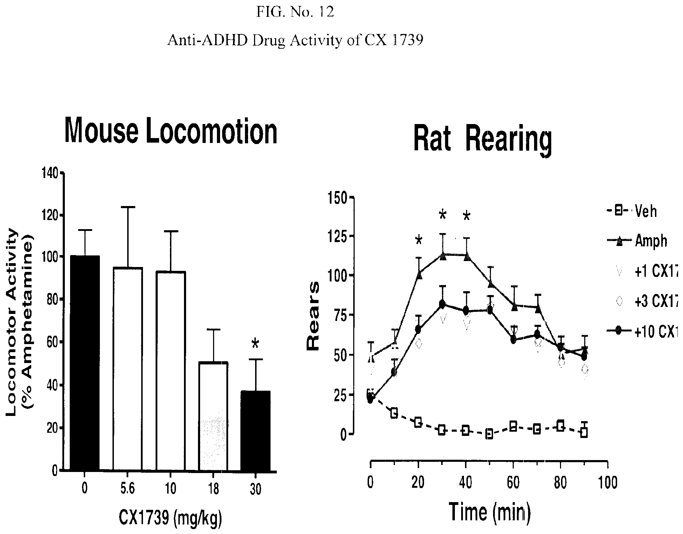

Both exemplary low impact ampakines CX717 and CX1739 were evaluated for their potential in abrogating amphetamine-stimulated locomotion in mice. Pre-treatment with CX717 or CX1739 inhibited amphetamine-stimulated locomotion in mice in a dose-dependent manner (Fig/s 11, 12). CX717 produces a significant decrease in amphetamine-stimulated locomotion activity at 3mg/kg and inhibits rearing at 1 and 3mg/kg (Figure 11), while CX1739 inhibited locomotion with 30 mg/kg ip producing a significant decrease in amphetamine-stimulated locomotion (Figure 12). In rats, CX1739 produced a modest reduction in amphetamine-stimulated rearing behavior at all doses tested.

Additional Novel Object Recognition behavioral model studies were conducted to further elucidate the anti-ADHD activities of CX717 and CX1739. Rats were habituated to a test box for two 5 minute sessions on consecutive days. Each test session consisted of two phases: in the first phase ("sample period"), two identical objects (A1 and A2) were placed in the far corners of the box, and the rats were allowed to explore the objects. After a 24 hour delay, rats were re-introduced to the arena ("test period") now containing a third identical copy of the familiar object (A3) and a new object (B), placed in the same locations as in the first phase, and the total time spent exploring each of the objects was determined over a 3-min duration. For CX717 drug treatment, rats were dosed 30 min prior to each sample session with either 0.1, 0.3, 1.0 mg/kg CX717 (p.o.); vehicle (0.5% methylcellulose, p.o.) or 0.9mg/kg Aricept (E2020, i.p.). The exploration times for the full 3-min were calculated and expressed as the discrimination index, d1, defined as the absolute difference in time spent exploring the novel object minus the familiar object.

For CX1739 Male Wistar rats were assessed for cognitive ability in a test apparatus comprising an open-field arena placed in a sound-attenuated room under dimmed lighting. Rat exploration and activity were captured by a digital camera, and viewed on a monitor in an adjoining area. Following a 5-minute habituation period, each animal was placed in the test arena in the presence of two identical objects (plastic shapes) and the time spent actively exploring the objects during a 5-minute test period (T1) was recorded. After 24 hours, each rat was again placed in the test arena for 5 minutes (T2) in the presence of one familiar object and one novel object, and the time spent exploring both objects was recorded. The Recognition Index was calculated as the percentage of total exploration time spent exploring the novel object during the retention session (T2), which provides the primary measure of cognitive function in this assay.

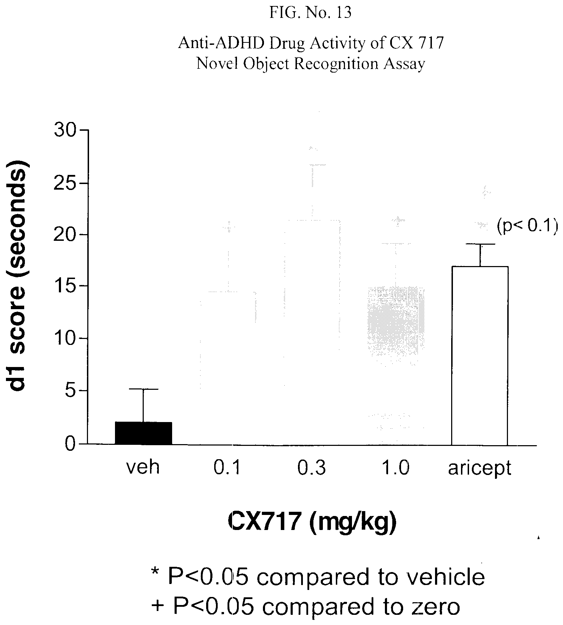

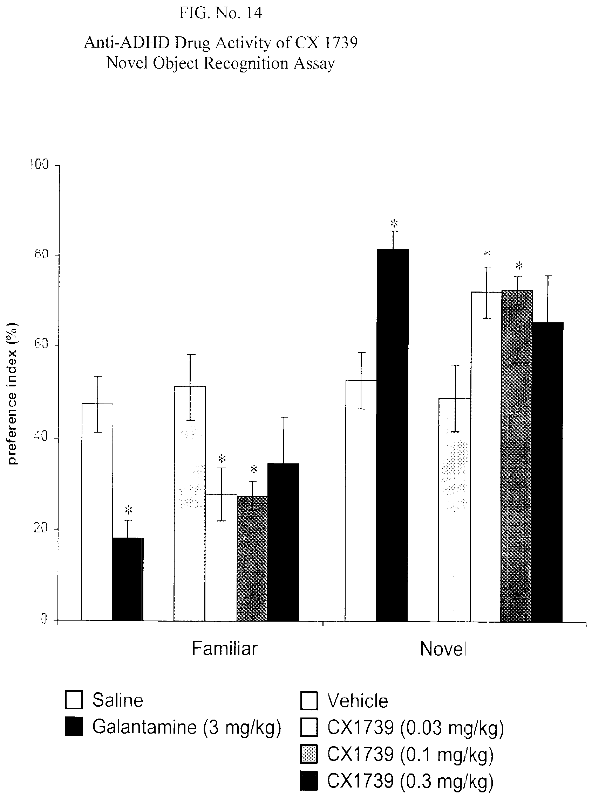

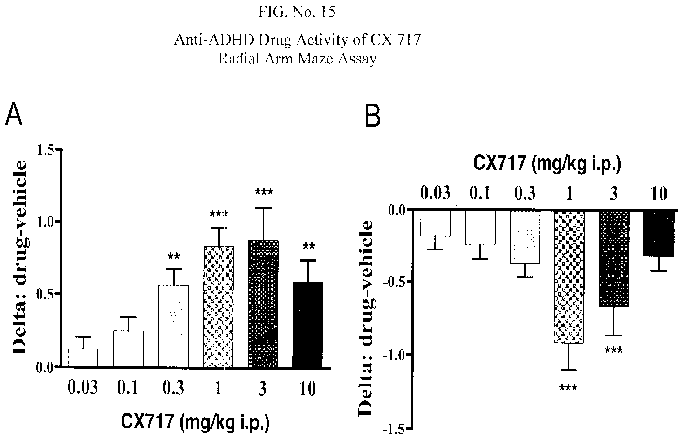

The Novel object recognition (NOR) paradigm is a rodent model of recognition learning memory retrieval and takes advantage of the spontaneous behavior of rodents to investigate a novel object compared with a familiar object. NOR has been employed extensively to indicate cognition-enhancing properties of drug candidates, predictive of therapeutic efficacy of positively-assaying drugs for reducing ADHD symptoms in humans. In mice treated with CX717 or Aricept (a positive control recognized as an effective drug for treating mild dementia), d1 scores increased with 0.3mg/kg CX717, indicating a significant increase compared to vehicle-treated mice (Figure 13). CX1739-treated mice also displayed a significant increase in preference for exploring the novel object (Figure 14). Critically, the minimal effective dose (MED) for CX1739 was 0.03mg/kg ip, demonstrating increased drug potency compared to CX717. 0.03mg/kg CX1739 also produced in increased preference for novel object exploration comparable to that of 3mg/kg galantamine, another positive control used to treat mild to moderate dementia (Figure 15).

Yet another animal model for evaluating ampakine drug activity employed herein is the well known and widely accepted Radial Arm Maze model. CX717 was tested for its ability to improve the performance of aged rats in an eight-arm radial maze using a cross-over design study. This behavioral test uses a standard eight-arm radial maze wherein five arms were open and baited with food pellets for the first morning session. Each animal was allowed to consume the food rewards present in the open arms and was then returned to its home cage. Six hours later animals were returned to the maze, wherein all eight- arms were open, but only the three arms previously closed contained food rewards. Scoring was based on the number of correct choices before an incorrect choice (re-entering an arm that was visited during the morning acquisition session) was made and on the total number of incorrect choices made before all three of the still baited arms were visited. CX717 was tested at six doses (0.03, 0.1, 0.3, 1, 3 and 10 mg/kg in hydroxypropyl β-cyclodextran, i.p.), administered 30 min prior to the start of the morning acquisition session and no drug was given prior to the second trial six hours later.

For CX1739 male Sprague-Dawley rats were trained to perform the win shift assay on the radial arm maze for approximately 10 weeks prior to the study. On each day of training and testing, each rat completed one trial, split into a sample phase and a test phase. During the sample phase, the rat was placed on a 12-arm radial arm maze surrounded by visual cues. Food wells at the end of each arm were baited with sucrose pellets but access to three arms, selected randomly, was blocked with a clear plastic divider at the arm entrance. The rat was allowed 5 minutes to search the maze and collect the available food pellets before being removed from the maze. Fifteen minutes later the rat was returned to the maze for the test phase of the trial. During the test phase, the rat was allowed access to all 12 arms of the maze but only the arms that had been blocked previously were baited with sucrose pellets. The rat was allowed 3 minutes to collect the available food pellets before being removed from the maze. On test days, rats were injected with CX1739 (0.03-1.0 mg/kg, i.p.) or vehicle immediately following the sample phase of the study. The primary dependent measure was the cumulative number of arm entries prior to entering the first, second, and third baited arm during the test phase.

CX717 produced a dose-dependent increase in the number of correct choices (before making an error) by the subjects in the second maze session and a decrease in the total number of errors. The difference in performance between drug and vehicle days was highly significant (Kruskal- Wallace test, P value = 0.008) for doses of 0.3 mg/kg and above (Wilcoxon Signed Rank Test) (Figure 15). These results demonstrate that CX717 potently enhances acquisition and retention of spatial memory in rats over the dose range of 0.3 to 10 mg/kg.

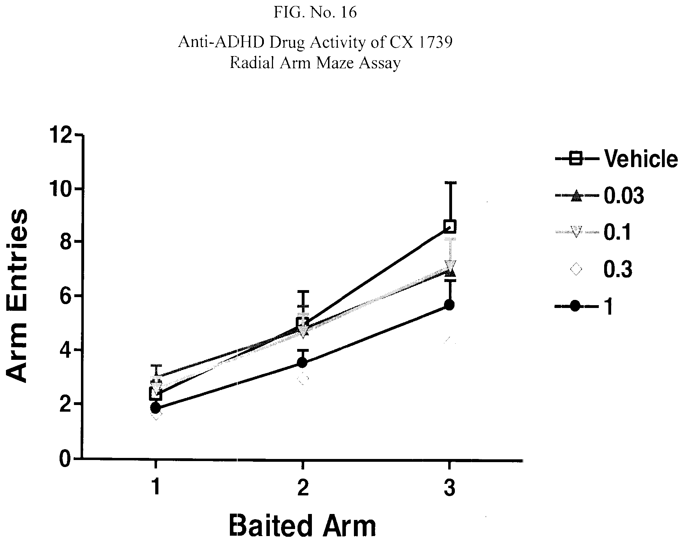

Rats treated with 0.3mg/kg CX1739 made significantly fewer entries before finding the third arm with a food pellet reward (p<0.05 using ANOVA followed by Bonferroni-corrected post-hoc test) also demonstrating pro-cognitive action of CX1739 at this dose (Figure 16).

Interestingly, the optimal dosages for CX717 and CX1739 in the foregoing maze assays were not the highest doses tested. Although study designs for the two exemplary low impact ampakines were slightly different, the dose producing the maximal effect for CX717 was observed to be somewhere between 1 and 3mg/kg ip while the optimal dose for CX1739 in reducing incorrect arm entries was found to be 0.3mg/kg (Figs. 15, 16).

Another useful animal model for evaluating ampakine activity is the Five Choice Serial Reaction Time Task in Rats. This assay requires rats to provide a single nose poke in response to a light stimulus presented in one of five locations within a 9-hole chamber. With a correct response a reward is delivered that the rat collects from the food magazine at the rear of the chamber. The withdrawal of its head from the food magazine initiates a short inter-trial interval followed by the presentation of the next randomly presented stimulus. An incorrect response results in a time out period (non-reinforcement) followed by the initiation of a new trial. Groups of 12 rats were used in this study, and all rats received all treatments using a randomized sequence. For evaluation using this model, CX1739 was selected as the exemplary low impact ampakine, administered at doses of 3 and 10 mg/kg i.p. 20 min prior to the start of the study.

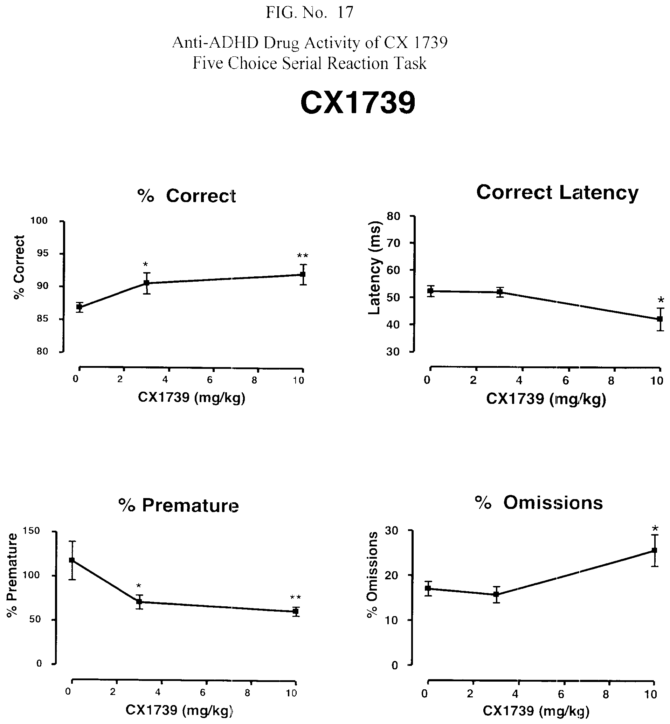

The 5CSRT task model has been proven to be sensitive to a wide range of compounds found to be clinically useful for treating ADHD. The instant examples demonstrate potent anti-ADHD activity of CX1739 in this highly predictive model. At doses of 3 and 10 mg/kg ip CX1739 significantly improved the accuracy of responding and reduced the number of premature responses in rats. At test dosing of 10mg/kg, CX1739 significantly reduced the latency to correct responses and produced a significant improvement in omissions (Figure 17).

Further elucidating the potent behavioral and cognitive effects of exemplary ampakines useful within the invention, additional studies using the well known Monkey Delayed Match to Sample assay were conducted. CX1739 was evaluated using aged Rhesus macaque monkeys (18 to 33 years old), undertaking the delayed match-to-sample (DMTS) task. The stimuli included red, blue, or yellow rectangles. A trial was initiated by presentation of a sample rectangle composed of one of the three colors. The sample rectangle remained in view until the monkey touched within its borders to initiate a preprogrammed delay (retention) interval. Following the delay interval, two choice rectangles were presented, with one of the two choice colors being the color matching the stimulus and the other (incorrect) color as one of the two remaining colors. A correct (matching) choice was reinforced. Non-matching choices were neither reinforced nor punished. The duration for each delay interval was adjusted for each subject until three levels of group performance accuracy approximated zero delay (85-100% of trials answered correctly), short delay interval (75-84% correct), medium delay interval (65-74% correct), and long delay interval (55-64% correct). The inter-trial interval was 5 seconds and each session consisted of 96 trials.

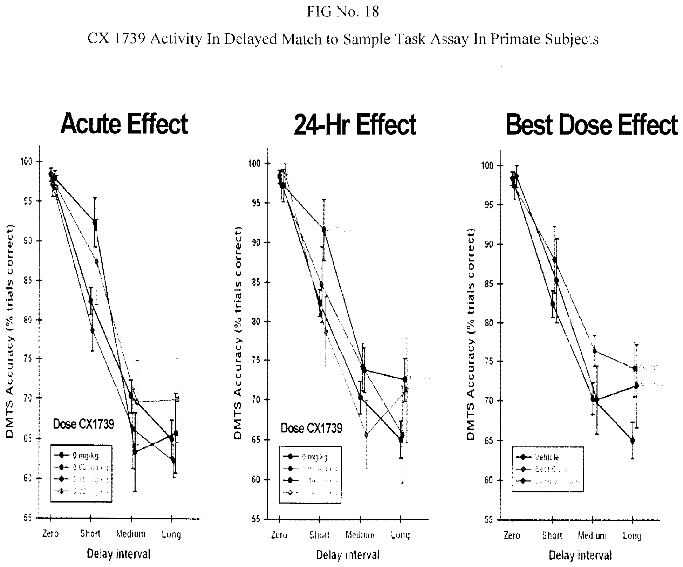

Oral administration of CX1739 45 min prior to testing increased mean accuracies during short delay trials relative to vehicle, particularly during sessions in which the two higher doses were administered. This pattern of improvement persisted in the sessions run 24 hours after CX1739 administration (Figure 18). CX1739 did not improve performance with medium or long delays.

The possibility that the actions of CX1739 might be specific for each individual monkey was examined by selecting a Best Dose for each subject. The Best Dose was based on the greatest overall (average of all four delay intervals) accuracy attained among the four doses tested. Administration of the Best Dose of CX1739 produced a statistically significant increase in task accuracy during sessions initiated 45 min after compound administration (F2,6=3.76, P=0.027; see Figure 4.2.5-1). With this Best Dose analysis, the increase in accuracy was restricted to long delay trials (t=2.42, P=0.017), an effect that appeared to carry over to sessions run 24 hours after compound administration (t=1.85, P=0.067). The memory retention curves associated with CX1739 treatment suggest that multiple components of memory could be affected.

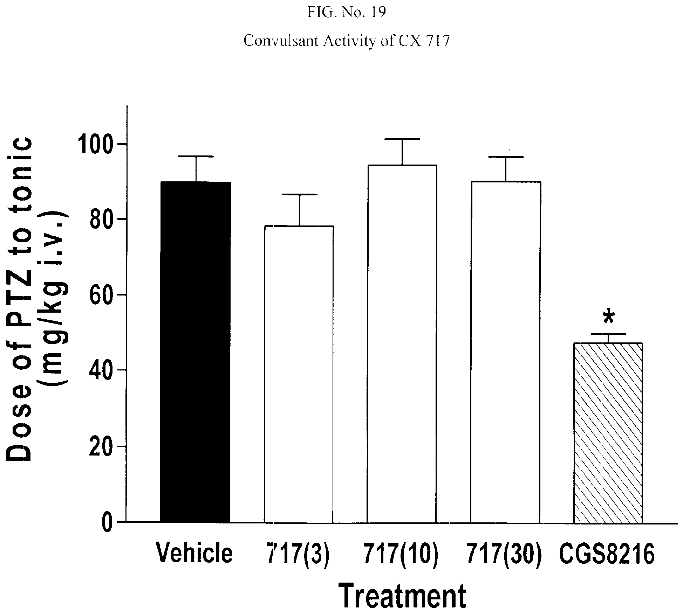

Follow on studies of CX717 and 1739 are continuing, employing additional well-known models for ADHD drug efficacy, coupled with a variety of ongoing safety studies. Preliminary safety studies already completed have included a range of toxicity studies focusing on critical convulsant risks widely observed for previously-studied ampakines. Using the well known PTZ Proconvulsant test, non-GLP safety studies were conducted to examine proconvulsant activity of CX717. Swiss Webster mice (n=12/group) were dosed with CX717 (3, 10 or 30 mg/kg p.o.), vehicle (0.5% methocel p.o.) or a positive control, CGS8216 (10 mg/kg in 70% PEG300: 30% water i.p.). Thirty minutes later, the GABA-A antagonist, pentylenetetrazol (PTZ) was infused in the tail vein until tonic seizures were observed. The time at which the first clonic and tonic seizure was observed was noted and used to determine the dose of PTZ necessary to induce both clonic and tonic seizure. These study showed that CX717, at doses of 30 mg/kg or less, did not decrease the dose of PTZ required to elicit a seizure, and thus was considered not to have proconvulsant activity in this model in the dosage range tested (Figure 19).

### Example V.

### Randomized, Double-Blind, Two-Period Crossover Human Clinical Trial Demonstrating Efficacy and Safety of the Exemplary Anti-ADHD Ampakine CX717 For Treating Adults with Attention-Deficit Hyperactivity Disorder

CX717 was used in the following clinical study demonstrating the effects of anti-ADHD ampakines within pharmaceutical compositions and therapeutic methods of the invention. In preclinical and clinical studies CX717 has been shown to improve alertness and ameliorate cognitive deficits, whereas the instant Example directly demonstrates that CX717 is a clinically effective drug for treating ADHD in human patients.

*This phase 2 study evaluated the efficacy, tolerability and safety of CX717 in the treatment of adult patients with ADHD. The study was designed and executed as a randomized, double-blind, multi-center, 2-period crossover study comparing 2 doses of CX717 (200 mg, or 800 mg, BID) with placebo. Each treatment period lasted 3 weeks with a 2-week washout in between. Subjects met DSM-IV criteria for adult ADHD and had moderate to severe symptoms. The primary efficacy measure was the change from baseline on the ADHD Rating Scale (ADHD-RS) with adult ADHD prompts. A total of 68 male subjects, who were 19 to 50 years old and met criteria of Adult ADHD as defined by DSM IV, were randomized. After accounting for early study dropouts, 51 subjects (75%) returned for efficacy assessments and completed both treatment periods (intent-to-treat population). In the repeated measures analysis at endpoint, treatment with CX717 800 mg BID was associated with superior efficacy compared to placebo on the total ADHD-RS (*p*=.002) and on both the inattentiveness (*p*=.027) and hyperactivity (*p*=.017) subscales. There were no statistically significant differences between the 200 mg BID dose and placebo on the same outcome measures. Treatment with CX717 was well tolerated with no clinically significant changes in cardiovascular and other safety parameters with either dose. Sleep disturbances and headache were the most frequently reported adverse events. This study demonstrates that CX717 800 mg BID is clinically effective for treatment of ADHD in humans, and generally well-tolerated.

Preparative to the instant investigation, phase 1 studies demonstrated safety and tolerability of CX717, as assessed by routine monitoring of vital signs (blood pressure and heart rate; supine and standing), routine 12-lead quantitative ECG (rhythm, ventricular rate, PR, QRS, QT and calculated QTc), continuous lead II ECG, laboratory investigation (hematology, serum biochemistry, coagulation, urinalysis, and urine microscopy) and routine collection of adverse events (AEs). In early healthy volunteers, CX717 has been determined to be well-tolerated with no serious AEs. CX717 is absorbed in a dose-proportional manner with a time-to-maximum concentration (tmax) of 4-5 hours and an elimination half-life (t1/2) of 9-10 hours, thus providing an opportunity for convenient once daily or twice daily dosing.

The present study further examined the clinical efficacy, tolerability and safety of CX717 as a drug treatment for adults with ADHD. Eligible subjects were randomized to 1 of 4 possible treatment sequences in which they received placebo (P) and either high dose (H) or low dose (L) of active treatment in random order (PL, PH, LP, and HP). Each treatment period was 3 weeks in duration with a 2-week washout in between. The study design was modeled after several similarly published ADHD studies with atomoxetine and stimulants, (Spencer et al.) with sample sizes in the mid 20s. Since this is the first such study with CX7171 in ADHD, it is assumed that efficacy would be achieved using historical assumptions in effect size, therefore, a total of 60 subjects were to be randomized.

All patients met DSM-IV criteria for adult ADHD, established by the Adult ADHD Clinical Diagnostic Scale (ACDS) Version 1.2. All patients gave written informed consent prior to enrollment and the conduct of the study was approved by either the International Review Board (IRB) at each site or by the central IRB. Each subject had at least moderately severe ADHD symptoms (ADHD-RS score of ≥22; Clinician's Global Impression of Severity [CGI-S] score of ≥4). Each subject was male, and between 18-50 years old, inclusively. Male patients were selected because the necessary reproductive toxicology studies that would have allowed women of childbearing potential to be included in the study had not been completed.

A subject was excluded from the study if he demonstrated more than a 25% change in total ADHD RS scores from screening to baseline. Subjects were excluded if they were taking medication specifically for treatment of ADHD symptoms (e.g., stimulants, atomoxetine, tricyclic antidepressants, or bupropion). ADHD treatments were discontinued (stimulants for 2 weeks and non-stimulant for 4 weeks) prior to the Period 1 baseline visit. Subjects were also excluded if they were taking antidepressants, anticonvulsants, or antipsychotics. Subjects were excluded from the study if they had a DSM-IV diagnosis of ADHD not otherwise specified, had a current or lifetime history of bipolar disorder or any psychotic disorder, had a current history of major depression, substance abuse or dependence, generalized anxiety disorder, obsessive compulsive disorder, panic disorder, or posttraumatic stress disorder, a history of epilepsy, seizures, syncope, unexplained blackout spell(s), head trauma with loss of consciousness, febrile seizures, or a currently active medical condition other than ADHD that could interfere with study participation.

CX717 capsules were administered orally as 200 mg BID or 800 mg BID doses. Matching placebo capsules were identical in appearance to the capsules containing active drug, and were administered orally. The study was divided into a series of periods: screening period - up to 4 weeks, treatment period 1 - 3 weeks; wash-out period - 2 weeks; treatment period 2 - 3 weeks; follow-up period - approximately 1 week.

Anti-ADHD drug efficacy was assessed using the Adult ADHD Rating Scale (DuPaul, 1998) with prompts (ADHD-RS) (Adler & Cohen, 2004). The primary efficacy outcome measure was the comparison of each dose of CX717 versus placebo on the change from baseline in ADHD symptoms as measured by the ADHD-RS at Week 3. Secondary efficacy parameters included comparisons of each dose of CX717 versus placebo for the ADHD-RS Inattentiveness Subscale Score of the ADHD-RS.

Safety was evaluated by adverse events (AEs), clinical laboratory tests, vital signs, physical examination, and electrocardiograms (ECGs).

The primary efficacy endpoint was the change from baseline in the ADHD-RS total score. Variables for the primary endpoint as well as for the secondary endpoint were based on within patient comparisons of high dose with placebo and low dose with placebo. The analyses treated subjects receiving high dose or placebo in either order as a unique entity and similarly, subjects who received low dose or placebo in either order as a unique group of its own. The primary efficacy analysis utilized the paired t-test. Descriptive statistics (number of observations, mean, standard deviation, median, minimum and maximum values) for the total score and the change from baseline were calculated by treatment group and visit and the last value was presented for both the intent-to-treat (ITT) and per protocol (PP) populations. All hypotheses testing for change from Baseline of scores, except testing for significant interaction, were performed at the 2-tailed 5% alpha significance level. P-values, based on 2-tailed tests, were rounded to 1 more decimal place than the standard deviation, up to a maximum of 3 decimal places; *p*-values <.001 were presented as '<.001' in all tables. A repeated measures analysis of ADHD-RS total score, and hyperactive and inattentiveness subscale scores was performed in addition to the planned analysis (secondary analyses). Baseline was defined as the last non-missing observation prior to dosing. Baseline for Period 1 was based on the last non-missing observation prior to the first dose date of that period recorded on the drug dispensing/accountability/adherence case report form page. Baseline for Period 2 was the last non-missing observation prior to first dose date of that period but not including any observations included in Period 1. Adverse events, laboratory parameters, ECG, and vital signs were described with summary statistics.

Sixty-eight subjects were randomized in this study. The number and percentage of subjects in active treatment groups are presented in Table 5.

**Table 5 - The number (%) of subjects in active treatment groups**

| **Disposition** | **Low Dose CX717 (n=33)** | **High Dose CX717 (n=35)** | **All Subjects (n=68)** |
|---|---|---|---|
| Randomized | 33 | 35 | 68 |
| Safety population* | 33 (100) | 32 (91) | 65 (96) |
| ITT population^{†} | 28 (85) | 23 (66) | 51 (75) |
| PP population^{‡} | 27 (82) | 22 (63) | 49 (72) |
| Early withdrawal^{§} | 5 (15) | 13 (37) | 18 (26) |

| | | | |
|---|---|---|---|
| * All subjects taking ≥1 dose of study medication ^{†} All subjects with ≥1 efficacy evaluation in both treatment periods ^{‡} All subjects completing both treatment periods ^{§} Withdrawal due to treatment emergent adverse event (TEAE; total - 3; 1 placebo, 2 high dose CX717); non-TEAE (1 - placebo); consent withdrawal (4 - 2 placebo, 2 high dose CX717); non-adherence (2 - 1 low dose CX717; 1 placebo); loss during follow-up (8 - 4 placebo; 1 low dose CX717; 3 high dose CX717) | | | |

In general, demographic characteristics were similar across the treatment sequences in the safety population, which included 65 of the subjects. Limitation of the study to male subjects was primarily to avoid reproductive toxicology studies that would have allowed women of childbearing potential to be included in the study. The study subjects had a mean age of 35.2 years old (median: 35.0; min-max: 19-50 years). Ninety-two percent of the subjects were white; 2% Asian, 6% other ethnicity/heritage. The percentage of subjects who were white was lower in the HP treatment sequence (80%) compared with the other 3 treatment sequences (94-100%). Similar demographic characteristics were observed for the ITT and PP populations. Other demographic characteristics at baseline are seen in Table 6.

**Table 6 - Baseline Demographic Characteristics of 65 Subjects in Safety Population**

| **Parameters** | **Study population** |
|---|---|
| Mean weight | 191.5 lbs |
| Mean height | 69.5 inches |
| Mean body mass index | 27.7 kg/m² |
| Percentage of subjects who had a childhood onset of ADHD prior to 7 years of age | 100 |
| Mean age at onset of ADHD | 5.7 years (min-max: 3-7; |
| | Range of means: 5.6 to 5.8 years) |
| Percentage of subjects who had combined ADHD subtype | 77% (50 subjects) |
| Percentage of subjects who had inattentive subtype | 23% (15 subjects) |
| Percentage of subjects who had hyperactive/impulsive subtype | 0% (0 subjects) |

In general, there were no remarkable differences across the treatment sequences with respect to the history of ADHD with the exception that the number of subjects with a combined ADHD subtype in the PL treatment sequence (16 subjects [100%]) was slightly higher than the number of subjects in the other treatment sequences (65% to 73%).

The primary efficacy endpoint was the change from baseline in the ADHD-RS total score. The mean change from baseline of the overall ADHD-RS total score for low dose group and the high dose group is represented in Figure 1. In the repeated measures analysis, a statistically significant treatment difference in favor of high dose CX717 compared to placebo was observed (p=.002). After 3 weeks, the mean change from baseline of ADHR-RS total score was -9.95 in the high dose group, and -5.96 in the placebo control group, with a significant difference of -4.73. The low dose did not differ from placebo.

Hyperactivity-Impusivity symptoms were analyzed using the ADHD-RS hyperactivity subscale in the ITT population, which paralleled those of the ADHD-RS total score. Greater improvement was observed for the high dose CX717 vs. placebo compared with the low dose CX717 vs. placebo, and the difference between high dose and placebo approached statistical significance at the last value (mean score: -5.57 vs. -2.78; p=.054 [mean difference = 3.00]), and was statistically significant at Week 3 (mean score: -5.68 vs. -2.78; p=.050 [mean difference = 3.18]) [[Fig. 2]. Differences between high dose CX717 vs. placebo were observed beginning at Week 1 (mean score: -4.22 vs. -2.22; p=.192 [mean difference = 2.00]). For the active dose vs. placebo comparison, a statistically significant difference was observed at the last value (mean score: -4.10 vs. -2.27; *p*=.046 [mean difference = 1.94]). The results of the PP population were similar to those for the ITT population with statistically significant differences between high dose CX717 vs. placebo observed at Week 3 and the last value (p*=*.050 at both time points). In the repeated measures analysis of ADHD-RS hyperactivity subscale there was a significant (*p*=.017) treatment effect for high dose compared with placebo. No difference was observed between low dose and placebo.

Treatment with CX717 200 mg or 800 mg BID for 3 weeks was generally safe and well tolerated in this study population of adults with ADHD. No deaths or serious adverse events occurred during the study. While the overall incidence of treatment-emergent adverse events (TEAEs) was higher for both doses of CX717 (low dose: 73%; high dose: 75%) compared with the placebo data (placebo low dose: 55%; placebo high dose: 56%), this difference did not reach statistical significance. This is the first demonstration in a human clinical study of therapeutic anti-ADHD activity for any ampakine, exemplified here by the low impact ampakine (CX717) shown to be effective in clinical treatment of adult patients with ADHD. While the low dose 200 mg BID did not differ from placebo, repeated measures analysis showed a statistically significant treatment effect in the 800 mg BID dose group for the ADHD-RS total score, as well as in the hyperactivity subscale scores. It should be emphasized that the magnitude of therapeutic effect observed in the present study is comparable to that reported for the amphetamine stimulants commonly used in these patients.

The foregoing Examples demonstrate profound and surprising advances in ADHD drug discovery employing low impact ampakines, provided herein. Glutamate is the major excitatory neurotransmitter in the brain, and neurotransmission, the communication between neurons, is principally mediated via glutamate acting on AMPA-type glutamate receptors. Ampakine compounds are positive allosteric modulators of the AMPA-type glutamate receptor that prolong the opening of the AMPA receptor-associated ion channel and facilitate or up-modulate the response to glutamate. Thus in the presence of ampakine molecules, neurotransmission is enhanced and communication within neuronal networks is improved. Ampakine molecules therefore have the potential for treating a wide range of central nervous system (CNS) disorders, including cognitive and psychiatric disorders.

The instant disclosure elucidates two general classes of ampakine compounds, termed "Low Impact" and "High Impact" ampakines. These molecules bind to different sites on the AMPA receptor complex, and while both molecules increase the activity of the AMPA receptor, they lead to different downstream effects in the brain. High impact ampakines bind to the AMPA receptor complex at the cyclothiazide-binding site, and stimulate the acute production of immediate early genes such as brain-derived neurotrophic factor (BDNF), a molecule essential for maintaining cell health and stabilizing new neuronal connections. Both classes of molecules improve learning and memory, but in addition, the high impact ampakine molecules may have disease-modifying activities. Yet, these added benefits are observed in conjunction with convulsions at elevated doses. The work product and attendant discoveries presented here demonstrate principal utility of low impact ampakines, exemplified by CX717 and CX1739, for mediating anti-ADHD effects with little or no convusant or preconvulsant activity at therapeutic dosage levels.

The exemplary ampakines described here, CX717 and CX1739, are surprisingly more potent than predecessor low impact cytokines described previously (exemplified by the failed cognitive drug candidate, CX516). CX717 and CX1739 both have significantly longer half-lives than CX516, and distinctly potentiate glutamatergic neurotransmission at clinically relevant doses. At the same time, these uniquely effective ampakines do not induce seizure potential activity, or change the dose of PTZ needed to induce tonic seizures. In contrast, CX614 and other high-impact ampakines alter AMPAR agonist binding and substantially increase BDNF production, while displays unacceptable convulsant activity at 16uM (a concentration that significantly increases BDNF production in hippocampal slices (Lauterborn et al, 2009).

Previous studies have shown that NGF and BDNF are upregulated as a consequence of intense neuronal activity, such as during a seizure (Gall and Isackson, 1989), which begs the question whether high-impact ampakines increase BDNF production by producing seizures, or whether their ability to increase BDNF production is due to their AMPAR-mediated up modulatory effects. These phenomena remain poorly understood.

A core discovery of the invention is that low-impact ampakines, exemplified by CX717 and CX1739, can be selected and characterized that exhibit little or no convulsant activity at significantly higher doses that previously contemplated. For CX1739 this unexpected low impact characteristic extends up to the limit of the drug's solubility (approximately 200uM), demonstrating that uniquely desirable pharmacological effects of ampakines for treating CNS disorders can be effectively differentiated from their adverse side effects.

The instant disclosure reveals that both CX717 and CX1739 increase the dentate gyrus fEPSPs in the hippocampus of anesthetized mice. Both molecules also exhibit activity to increase LTP, which is widely considered to be a cellular mechanism underlying learning and memory. This augmented memory is reflected in positive results from the eight-arm maze (short term memory) tasks. The combined discoveries herein are surprising, even in view of prior reports of certain ampakines enhancing performance in selected cognitive, memory and learning assays (see, eg., Staubli et al., 1994a; Shors et al., 1995; Rogan et al., 1997).

Beyond the novel therapeutic uses of CX717 and CX1793 for treating ADHD and related conditions, described here, these low impact ampakines are also positive drug candidates for managing schizophrenia. Reductions in AMPA receptor protein are associated with schizophrenia histology. Assayed tissue samples from schizophrenics and matched controls reveal that Glur2 mRNA of the flip and flop variant are reduced in the hippocampal formation of schizophrenics (Eastwood et al, 1997). Thus, enhancement of AMPAR-mediated signaling of those with diminished AMPAR activity should alleviate common symptoms of schizophrenics. CX516 failed to enhance use of language, attention and overall executive functioning in schizophrenic patients. However, in view of the surprising discoveries here, of high potency, enduring half life and low convulsant activity of CX717 and CX1739, these distinctly useful ampakines are qualified clinical drug candidates for treating and managing schizophrenia in addition to ADHD.

All publications and patents cited herein are incorporated herein by reference for the purpose of describing and disclosing, for example, the materials and methodologies that are described in the publications which may be used in connection with the presently described invention. The publications discussed above and throughout the text are provided solely for their disclosure prior to the filing date of the present application. Nothing herein is to be construed as an admission that the inventors are not entitled to antedate such disclosure by virtue of prior invention.

## Claims

1. A composition comprising one or more ampakine for use in treating an attention deficit disorder (ADD) in a mammalian subject, the use comprising administering to the subject the one or more ampakines in an amount effective to alleviate one or more symptom(s) of ADD in the subject; wherein the one or more ampakine is selected from:
1-(benzofurazan-5-ylcarbonyl)morpholine (CX717); and
N-methyl-N-(tetrahydro-2H-pyran-4-yl)-[2,1,3]-benzoxadiazole-5-carboxamide (CX1739);
and wherein when the ampakine is CX717 the amount effective to alleviate one or more symptom(s) of ADD in the subject is a minimal effective daily dose greater than 400 mg and up to about 1600 mg.

2. A composition for use according to claim 1, wherein when the ampakine is CX717 the amount effective to alleviate one or more symptom(s) of ADD in the subject is a daily dose of about 1600 mg.

3. A composition for use according to claim 1, wherein when the ampakine is CX717 the amount effective to alleviate one or more symptom(s) of ADD in the subject is a daily dose of about 1600 mg, administered as about 800 mg twice daily (BID).

4. A composition comprising one or more ampakine for use according to any one of claims 1-3, wherein the attention deficit disorder is attention deficit hyperactivity disorder (ADHD), optionally
wherein the ADHD is Attention Deficit Hyperactivity Disorder-predominantly hyperactive-impulsive subtype, Attention Deficit Hyperactivity Disorder-predominantly inattentive subtype, or Attention Deficit Hyperactivity Disorder-combined subtype.

5. A composition comprising one or more ampakine for use according to any one of claims 1-3, wherein the attention deficit disorder is Conduct Disorder or Oppositional Defiant Disorder, or wherein the subject is a human child, adolescent, or adult.

6. A composition comprising one or more ampakine for use according to claim 1, wherein the anti-ADD ampakine is formulated or administered with a secondary therapeutic agent.

7. A composition comprising one or more ampakine for use according to claim 6, wherein the secondary therapeutic agent is a secondary anti-ADHD drug.

8. A composition comprising one or more ampakine for use according to claim 7, wherein the secondary anti-ADHD drug is a stimulant anti-ADHD drug selected from methylphenidate, methamphetamine, amphetamine, dextroamphetamine and lisdexamfetamine.

9. A composition comprising one or more ampakine for use according to claim 7, wherein the stimulant anti-ADHD drug is administered at a sub-therapeutic dose in a combinatorial formulation or coordinate administration protocol with the anti-ADHD ampakine, yielding a therapeutically effective combined formulation or coordinate protocol, with lower stimulant and other adverse side effects compared to a therapeutic dose of the stimulant anti-ADHD drug alone.

10. A composition comprising one or more ampakine for use according to claim 7, wherein the secondary anti-ADHD drug is a non-stimulant anti-ADHD drug selected from atomoxetine, clonidine and guanfacine. J

11. A composition comprising one or more ampakine for use according to claim 6, wherein the secondary therapeutic agent is a drug selected to treat a co-morbid condition, selected from a psychotic, antidepressant, anti-convulsant, mood-stabilizer, anxiolytic, benzodiazepine, calcium channel blocker, or anti-inflammatory drug, and combinations thereof.

12. A composition comprising one or more ampakine for use according to claim 11, wherein the antipsychotic drug is selected from aripiprazole, ziprasidone, risperidone, quetiepine, or olanzapine.

13. A composition comprising one or more ampakine for use according to claim 11, wherein the antidepressant drug is selected from tri-cyclic antidepressants (TCAs), specific monoamine reuptake inhibitors, selective serotonin reuptake inhibitors, selective norepinephrine or noradrenaline reuptake inhibitors, selective dopamine reuptake inhibitors, norepinephrine-dopamine reuptake inhibitors, serotonin-norepinephrine reuptake inhibitors, multiple monoamine reuptake inhibitors, monoamine oxidase inhibitors, and atypical antidepressants.

14. A composition comprising one or more ampakine for use according to claim 6, wherein the secondary therapeutic agent is an anti-convulsant drug.

15. A composition comprising one or more ampakine for use according to claim 6, wherein the secondary therapeutic agent is an anxiolytic agent.

## Patentansprüche

1. Eine Zusammensetzung, umfassend ein oder mehrere Ampakine zur Verwendung bei der Behandlung einer Aufmerksamkeitsdefizitstörung (ADS) bei einem Säugetier, wobei die Verwendung die Verabreichung des einen oder der mehreren Ampakine an das Subjekt in einer therapeutisch wirksamen Menge umfasst, die geeignet ist, ein oder mehrere Symptome der ADS bei dem Subjekt zu lindern; wobei das eine oder die mehreren Ampakine ausgewählt sind aus:
1-(Benzofurazan-5-ylcarbonyl)morpholin (CX717); und
N-Methyl-N-(tetrahydro-2H-pyran-4-yl)-[2,1,3]benzoxadiazol-5-carboxamid (CX1739);
und wobei, wenn das Ampakin CX717 ist, die zur Linderung eines oder mehrerer Symptome einer ADS bei dem Subjekt einer minalen wirksamen Tagesdosis von mehr als 400 mg bis etwa 1600 mg entspricht.

2. Eine Zusammensetzung zur Verwendung nach Anspruch 1, wobei, wenn das Ampakin CX717 ist, die zur Linderung eines oder mehrerer Symptome einer ADS bei dem Subjekt wirksamen Tagesdosis von etwa 1600 mg entspricht.

3. Eine Zusammensetzung zur Verwendung nach Anspruch 1, wobei, wenn das Ampakin CX717 ist, die zur Linderung eines oder mehrerer Symptome einer ADS bei dem Subjekt wirksamen Tagesdosis von etwa 1600 mg entspricht, die zweimal täglich (BID) in einer Dosis von jeweils etwa 800 mg verabreicht wird.

4. Eine Zusammensetzung, umfassend ein oder mehrere Ampakine zur Verwendung nach einem der Ansprüche 1-3, wobei es sich bei der Aufmerksamkeitsdefizitstörung um eine Aufmerksamkeitsdefizit-Hyperaktivitätsstörung (ADHS) handelt, wobei die ADHS eine Aufmerksamkeitsdefizit-Hyperaktivitätsstörung der vorwiegend hyperaktiv-impulsiven Unterkategorie, die Aufmerksamkeitsdefizit-Hyperaktivitätsstörung der vorwiegend unaufmerksamen Unterkategorie oder die Aufmerksamkeitsdefizit-Hyperaktivitätsstörung der kombinierten Unterkategorie ist.

5. Eine Zusammensetzung, umfassend ein oder mehrere Ampakine, zur Verwendung nach einem der Ansprüche 1-3, wobei die Aufmerksamkeitsdefizitstörung eine Verhaltensstörung oder eine oppositionelle Trotzstörung ist oder wobei das Subjekt ein menschliches Kind, ein Jugendlicher oder Erwachsener ist.

6. Eine Zusammensetzung, umfassend ein oder mehrere Ampakine zur Verwendung nach Anspruch 1, wobei das Ampakin gegen ADS zusammen **mit** einem sekundären therapeutischen Mittel formuliert oder verabreicht wird.

7. Eine Zusammensetzung, umfassend ein oder mehrere Ampakine zur Verwendung nach Anspruch 6, wobei das sekundäre therapeutische Mittel ein sekundäres Arzneimittel gegen ADHS ist.

8. Eine Zusammensetzung, umfassend ein oder mehrere Ampakine zur Verwendung nach Anspruch 7, wobei das sekundäre Arzneimittel gegen ADHS ein stimulierendes Arzneimittel gegen ADHS ist, ausgewählt aus Methylphenidat, Methamphetamin, Amphetamin, Dextroamphetamin und Lisdexamfetamin.

9. Eine Zusammensetzung, umfassend ein oder mehrere Ampakine zur Verwendung nach Anspruch 7, wobei das stimulierende Arzneimittel gegen ADHS in einer subtherapeutischen Dosis in einer Kombinationsformulierung oder im Rahmen eines abgestimmten Verabreichungsschemas zusammen mit dem Ampakin gegen ADHS verabreicht wird, wodurch eine therapeutisch wirksame Kombinationsformulierung oder ein therapeutisch wirksames abgestimmtes Verabreichungsschema bereitgestellt wird, die bzw. das im Vergleich zu einer einzigen therapeutischen Dosis des stimulierenden Arzneimittels gegen ADHS geringere stimulanzienbedingte und andere unerwünschte Nebenwirkungen aufweist.

10. Eine Zusammensetzung, umfassend ein oder mehrere Ampakine zur Verwendung nach Anspruch 7, wobei das sekundäre Arzneimittel gegen ADHS ein nicht-stimulierendes Arzneimittel gegen ADHS ist, ausgewählt aus Atomoxetin, Clonidin und Guanfacin.

11. Eine Zusammensetzung, umfassend ein oder mehrere Ampakine zur Verwendung nach Anspruch 6, wobei das sekundäre therapeutische Mittel ein Arzneimittel ist, das zur Behandlung einer Begleiterkrankung ausgewählt ist und aus Psychopharmaka, Antidepressiva, Antikonvulsiva, Stimmungsstabilisatoren, Anxiolytika, Benzodiazepinen, Calciumkanalblockern oder entzündungshemmenden Arzneimitteln sowie Kombinationen davon ausgewählt ist.

12. Eine Zusammensetzung, umfassend ein oder mehrere Ampakine zur Verwendung nach Anspruch 11, wobei das Antipsychotikum aus Aripiprazol, Ziprasidon, Risperidon, Quetiapine oder Olanzapin ausgewählt ist.

13. Eine Zusammensetzung, umfassend ein oder mehrere Ampakine zur Verwendung nach Anspruch 11, wobei das Antidepressivum ausgewählt ist aus trizyklischen Antidepressiva (TZA), spezifischen Monoamin-Wiederaufnahmehemmern, selektiven Serotonin-Wiederaufnahmehemmern, selektiven Norepinephrin- oder Noradrenalin-Wiederaufnahmehemmern, selektiven Dopamin-Wiederaufnahmehemmern, Norepinephrin-Dopamin-Wiederaufnahmehemmern, Serotonin-Norepinephrin-Wiederaufnahmehemmer, Mehrfach-Monoamin-Wiederaufnahmehemmer, Monoaminoxidasehemmer und atypischen Antidepressiva.

14. Eine Zusammensetzung, umfassend ein oder mehrere Ampakine zur Verwendung nach Anspruch 6, wobei das sekundäre therapeutische Mittel ein Antikonvulsivum ist.

15. Eine Zusammensetzung, umfassend ein oder mehrere Ampakine zur Verwendung nach Anspruch 6, wobei das sekundäre therapeutische Mittel ein Anxiolytikum ist.

## Revendications

1. Une composition comprenant une ou plusieurs ampakines destinée à être utilisée pour traiter un trouble de déficit de l'attention (TDA) chez un sujet mammifère, l'utilisation comprenant l'administration au sujet d'une ou de plusieurs ampakines en quantité efficace pour soulager un ou plusieurs symptôme(s) de TDA chez le sujet ; dans laquelle la ou les ampakines sont choisies parmi :
1-(benzofurazan-5-ylcarbonyl)morpholine (CX717) ; et
N-méthyl-N-(tétrahydro-2H-pyran-4-yl)-[2,1,3]-benzoxadiazole-5-carboxamide (CX1739) ;
et dans laquelle, lorsque l'ampakine est une CX717, la quantité efficace pour soulager un ou plusieurs symptôme(s) de TDA chez le sujet est une dose effective quotidienne minimum supérieure à 400 mg et pouvant aller jusqu'à 1600 mg.

2. Une composition destinée à être utilisée selon la revendication 1, dans laquelle, lorsque l'ampakine est une CX717, la quantité efficace pour soulager un ou plusieurs symptôme(s) de TDA chez le sujet est une dose quotidienne de près de 1600 mg.

3. Une composition destinée à être utilisée selon la revendication 1, dans laquelle, lorsque l'ampakine est une CX717, la quantité efficace pour soulager un ou plusieurs symptôme(s) de TDA chez le sujet est une dose quotidienne de près de 1600 mg, administrée sous forme d'environ 800 mg deux fois par jours (BID).

4. Une composition comprenant une ou plusieurs ampakines destinée à être utilisée selon l'une quelconque des revendications 1-3, dans laquelle le trouble de déficit de l'attention est un trouble de déficit de l'attention avec hyperactivité (TDAH), facultativement dans laquelle le TDAH est un Trouble de Déficit de l'Attention avec Hyperactivité - sous-type à dominante hyperactive-impulsive, un Trouble de Déficit de l'Attention avec Hyperactivité - sous-type à dominante inattentive ou un Trouble de Déficit de l'Attention avec Hyperactivité - sous-type combiné.

5. Une composition comprenant une ou plusieurs ampakines destinée à être utilisée selon l'une quelconque des revendications 1-3, dans laquelle le trouble de déficit de l'attention est un Trouble des Conduites, ou un Trouble Oppositionnel avec Provocation, ou dans laquelle le sujet est un enfant, adolescent ou adulte humain.

6. Une composition comprenant une ou plusieurs ampakines destinée à être utilisée selon la revendication 1, dans laquelle l'ampakine anti-TDA est formulée ou administrée avec un agent thérapeutique secondaire.

7. Une composition comprenant une ou plusieurs ampakines destinée à être utilisée selon la revendication 6, dans laquelle l'agent thérapeutique secondaire est un médicament anti-TDAH secondaire.

8. Une composition comprenant une ou plusieurs ampakines destinée à être utilisée selon la revendication 7, dans laquelle le médicament anti-TDAH secondaire est un médicament anti-TDAH stimulant choisi parmi le méthylphénidate, la méthamphétamine, l'amphétamine, la dextroamphétamine et la lisdexamfétamine.

9. Une composition comprenant une ou plusieurs ampakines destinée à être utilisée selon la revendication 7, dans laquelle le médicament anti-TDAH stimulant est administré en dose sous-thérapeutique sous forme de formulation combinatoire ou de protocole d'administration coordonnée avec l'ampakine anti-TDAH, donnant lieu à une formulation combinée thérapeutiquement efficace ou à un protocole coordonné, avec un effet stimulant et autres effets secondaires indésirables plus bas par rapport à ceux d'une dose thérapeutique du médicament anti-TDAH stimulant seul.

10. Une composition comprenant une ou plusieurs ampakines destinée à être utilisée selon la revendication 7, dans laquelle le médicament anti-TDAH secondaire est un médicament anti-TDAH non stimulant choisi parmi l'atomoxétine, la clonidine et la guanfacine.

11. Une composition comprenant une ou plusieurs ampakines destinée à être utilisée selon la revendication 6, dans laquelle l'agent thérapeutique secondaire est un médicament choisi pour traiter une comorbidité, choisi parmi un antipsychotique, un antidépresseur, un anti-convulsant, un stabilisateur d'humeur, un anxiolytique, une benzodiazépine, un inhibiteur des canaux calciques, ou un anti-inflammatoire, et des combinaisons de ceux-ci.

12. Une composition comprenant une ou plusieurs ampakines destinée à être utilisée selon la revendication 11, dans laquelle le médicament antipsychotique est choisi parmi l'aripiprazole, la ziprasidone, la rispéridone, la quétiapine ou l'olanzapine.

13. Une composition comprenant une ou plusieurs ampakines destinée à être utilisée selon la revendication 11, dans laquelle le médicament antidépresseur est choisi parmi les antidépresseurs tricycliques (ATCs), les inhibiteurs spécifiques de la recapture des monoamines, les inhibiteurs sélectifs de la recapture de la sérotonine, les inhibiteurs sélectifs de la recapture de la norépinéphrine ou noradrénaline, les inhibiteurs sélectifs de la recapture de la dopamine, les inhibiteurs de la recapture de la norépinéphrine-dopamine, les inhibiteurs de la recapture de la sérotonine-norépinéphrine, les inhibiteurs multiples de la recapture des monoamines, les inhibiteurs de la monoamine-oxydase, et les antidépresseurs atypiques.

14. Une composition comprenant une ou plusieurs ampakines destinée à être utilisée selon la revendication 6, dans laquelle l'agent thérapeutique secondaire est un médicament anti-convulsant.

15. Une composition comprenant une ou plusieurs ampakines destinée à être utilisée selon la revendication 6, dans laquelle l'agent thérapeutique secondaire est un agent anxiolytique.
